# EUROPEAN PATENT SPECIFICATION

(11) **EP 1 495 116 B1**
(45) Date of publication and mention of the grant of the patent: **30.05.2012**
(21) Application number: 03745058.2
(22) Date of filing: 21.03.2003
(51) Int. Cl.: C12N 9/16

(54) **SACCHAROMYCES CEREVISIAE YEAST STRAIN WITH IMPROVED PHYTASE ACTIVITY**
SACCHAROMYCES-CEREVISIAE-HEFESTAMM MIT VERBESSERTER PHYTASEAKTIVITÄT
SOUCHE DE LEVURE SACCHAROMYCES CEREVISIAE A ACTIVITE DE LA PHYTASE AMELIOREE

(30) Priority: 22.03.2002 SE 0200911; 26.08.2002 US 228785
(43) Date of publication of application: 12.01.2005
(73) Proprietor: EDO Biotech AB, 437 32 Lindome (SE)
(72) Inventor: SANDBERG, Ann-Sofie, S-431 38 Mölndal (SE); ANDLID, Tomas, S-418 71 Göteborg (SE)
(74) Representative: Inger, Lars Ulf Bosson
(86) International application number: PCT/SE2003/000476
(87) International publication number: WO 2003/080826

(56) References cited:
- EP-A1- 0 649 600
- WO-A1-01/29073
- WO-A1-01/36607
- US-A- 5 834 286
- MOORE ELIZABETH ET AL.: 'Molecular cloning, expression and evaluation of phosphohydrolases for phytate-degrading activity' JOURNAL OF INDUSTRIAL MICROBIOLOGY vol. 14, 1995, pages 396 - 402, XP000575066
- TURK MARIA ET AL.: 'Inositol hexaphosphate hydrolysis by Baker's yeast. Capacity, kinetics and degradation products' J. AGRIC. FOOD CHEM. vol. 48, 2000, pages 100 - 104, XP002966589
- SHNYREVA M.G. ET AL.: 'Biochemical properties and excretion behavior of repressible acid phosphatases with altered subunit composition.' MICROBIOL. RES. vol. 151, 1996, pages 291 - 300, XP002903093
- FASCHER K.D. ET AL.: 'Role of trans-activating proteins in the generation of active chromatin at the PHO5 promoter in S. cerevisiae' EMBO J. vol. 9, no. 8, 1990, pages 2523 - 2528, XP002903081
- NOBUO OGAWA ET AL.: 'New components of a system for phosphate accumulation and polyphosphate metabolism in saccharomyces cerevisiae revealed by genomic expression analysis' MOLECULAR BIOLOGY OF THE CELL vol. 11, December 2000, pages 4309 - 4321, XP002967061
- YASUJI OSHIMA ET AL.: 'Regulation of phosphatase synthesis in saccharomyces cerevisiae - a review 1' GENE vol. 179, 1996, pages 171 - 177, XP004071980
- YANMING HAN ET AL.: 'Expression of an aspergillus niger phytase gene (phyA) in saccharomyces cerevisiae' APPLIED AND ENVIRONEMENTAL MICROBIOLOGY vol. 65, no. 5, May 1999, pages 1915 - 1918, XP002118710

## Description

### BACKGROUND OF THE INVENTION

The present invention relates to a phytase active yeast, in particular a modified *Saccharomyces cerevisiae* used for fermentation purposes, fermented products after fermentation using said modified *Saccharomyces cerevisiae,* the use of phytase obtained using said strain, as well as derived inositol phosphate derivatives including myoinositol.

Iron deficiency is one of the most common nutrition disorders worldwide. In addition to affecting a large proportion of infants, children and women in the developing world, iron deficiency is the only nutrient deficiency of significant prevalence in all developed nations as well. Deficiency of iron and probably zinc are highly prevalent in developing countries. where the diet is based on cereals and legumes but also in vulnerable population groups, in industrialized countries, with high requirements such as women or fertile age, infants and adolescents. In developing countries iron deficiency, due to poor bioavailability, retards normal brain development in infants and effects the success of a pregnancy by increasing premature deliveries, as well as morbidity of mother and child at or around child-birth. Zinc deficiency prevents normal child growth and greatly weakens the immune system leading to more infections. The body requires a high amount or calcium during growth. The amount of bone in later years is determined by the starting amount (the peak bone mass) and it's subsequent loss. Both are directly relevant for the subsequent development of osteoporosis.

Mild to moderate iron deficiency is often not recognized, but nevertheless may affect poorly defined parameters such as normal vigour, physical and mental endurance, and quality of life.

Iron deficiency is caused primarily by chronic blood loss. Using approximate values, inescapable loss of iron in sweat and cellular desquamation amounts to 1-2 mg per day. This is readily balanced in men by the absorption of 10% of the average dietary intake of 10-15 mg daily. In women of fertile ages. however, menstrual bleeding adds another 1 mg to the daily loss, making it more difficult to achieve an adequate balance.

Several haematologic and biochemical tests are well established for screening or diagnosis of iron deficiency individuals as well as for population based assessment.

The amount of iron absorbed from the diet at any one time is dependent on three factors: the quantity of iron, the composition of the diet and the behavior of the mucosa of the upper small bowel. Variation in the bioavailability (the portion of total iron in the diet absorbed and utilized by the organism) of food iron are of greater importance for iron nutrition than is the amount of iron in the diet. The haem iron in meat, poultry and fish is easily absorbed whatever the dietary composition whereas non-heme iron is markedly influenced by other ingredients in the diet. A number of promoters and inhibitors of iron absorption have been identified. The bioavailability of the iron in any particular diet ultimately depends on the relative quantities of promoters and inhibitors of iron absorption present in that diet.

Although the element is the second most abundant metal in the earth's crust, its low solubility makes its acquisition for metabolic use a major challenge. Most environmental iron exists as insoluble salts. Gastric acidity assists the conversion to absorbable forms, but the efficiency of this process is limited. Many plants produce powerful chelators, such as the phytate (organic polyphosphate), which are potent inhibitors of iron absorption found in e.g., cereals and legumes.

Zinc is a component of more than 300 enzymes needed to repair wounds, maintain fertility in adults and growth in children, synthesize protein, help cells reproduce, preserve vision, boost immunity, and protect against free radicals, among other functions.

Zinc deficiency is common in individuals or populations whose diets are low in sources of readily bioavailable zinc, such as meat, and high in unrefined cereals that are rich in phytate, a diet common in the poorer areas of the world. Vegetarian or lacto-vegetarian diets overall result in high intake of phytate, which is accompanied by a greater risk of zinc deficiency. Zinc deficiency in human caused by nutrition was first described in adolescent boys and girls in Egypt and Iran in the late 1960:s and early 1970's. The symptoms were severe growth failure, hypogonadism with delayed sexual maturation. The cause of the deficiency was a diet based on unleavened wholemeal bread with a high content of phytate and low intake of animal protein. Treatment with zinc and an adequate intake of other nutrients improved growth and sexual maturation. Dietary zinc deficiency has later been described among the children of many countries, such as Turkey, China and Yugoslavia. Another group at risk are pregnant women.

Other clinical manifestations of zinc deficiency than mentioned above are suppressed immunity, poor heating, dermatitis and impairments in neuropsychological functions.

The diagnosis of zinc deficiency is a problem while sensitive indices of zinc status are lacking. The most widely used indices of zinc status are levels of zinc in plasma or serum. These parameters may be decreased in cases of severe and moderate deficiency. Dietary data and indirect measures of bone health indicate that the bioavailability of calcium is important when habitual intakes are low, especially during periods of bone growth or loss. Bioavailability of calcium was found to be low in diets high in unrefined cereals, that are rich in phytate.

Cereals, together with oil seeds and legumes, supply a majority of the dietary protein, calories, vitamins, and minerals to the bulk population. Phosphorous, potassium, magnesium, calcium and traces of iron and other minerals are found in cereals. Barley and wheat provide 50 and 36 mg Ca/100 g respectively. Barley provides 6 mg of iron per 100 g; millet provides 6.8; oats. 4.6 and wheat, 3.1.

Inositol hexaphosphate. IP6. is ubiquitous in nature and comprises the bulk of eukaryotic cell inositol phosphate content. In plants, IP6 constitutes the principal storage form of phosphorous, in particular whole grains of cereals and legumes are rich in IP6. In cereals phytate is located in bran and germ, whereas in legume seeds phytate occurs in the protein bodies in the endosperm. The highly negatively charged IP6 forms various complexes with minerals and proteins, commonly known as phytate. Phytate is considered an anti-nutrient due to the formation of precipitated complexes that strongh reduces the absorption of essential dietary minerals such as iron, zinc, calcium and magnesium. A dose dependent inhibition of iron, zinc and calcium absorption by phytate has been demonstrated in humans. Moreover, inositol penta phosphate has been identified as an inhibitor of iron and zinc absorption. In addition, it has been suggested that IP6 may influence negatively on solubility, digestibility and activity of proteins such as digesting enzymes Reddy et al. Reduction in antinutritional and toxic components in plant foods by fermentation. Food Res. Int. 27. 281-290. Degradation of phytate to low levels in cereal and legume meals was demonstrated to markedly improve iron and zinc absorption in humans. To improve iron absorption the degradation has to be virtually complete. Thus. once phytate is degraded these foods become a good source of dietary minerals.

Degradation of phytate is possible with phytases. Native phytase in some cereals may be active during traditional processing such as soaking, germination, matting and fermentation and phytate degradation can be improved by process optimization, selection of specific starter cultures, or phytase can be added. It has also been demonstrated that phytate degradation in the stomach and small intestine of humans occurs as a result of activity of dietary phytase of plant or microbial origin thereby improving iron and zinc absorption. Consumption of foods containing active phytase enzymes is therefor an alternative to phytate removal during food processing. The plant phytase is less stable than the microbial phytase at the physiological conditions of the gastro-intestine and is therefor less effective in this respect.

Different feedstuffs having an improved phytase activity are available containing phytase derived from Aspergillus species, in order to reduce the need of inorganic phosphorous addition, thereby reducing overload of phosphorous present in faeces from animals. (e.g., EP-B1-0649 600).

Since IP6 is such a common compound in nature some microorganisms would be expected to have the ability to degrade IP6 and utilize the hydrolyzed phosphorous. This is true for certain bacteria. Riedereret al. (1991) Removal of N-glycosylation sites of the yeast acid phosphatase severely affects protein folding J. Bacteriol. 173. 3539-3546 and many fungi. Shieh et al. (1968) Survey of microorganism for the production of extracellular phytase. Appl. Microbiol. 16, 1348-1351: Dvoráková et al. (1997) Characterization of phytase produced by Aspergillus niger. Folia Microbiol. 42. 349-352: Wyss et al. (1998) Comparison of the thermostability properties of three acid phosphatases from molds: Aspergillus fumigatus phytase. A. niger phytase, and A. niger pH 2.5 acid phosphatase. Appl. Environ. Microbiol. 64. 4446-4451 that are well known to synthesize secretory so called phytases, i.e. phosphatases hydrolyzing IP6 to inositol pentaphosphate (IP5) and inorganic ortho-phosphate (Pᵢ). Phytases, derived from Aspergillus sp are frequently used in animal feeds to improve phosphorous and mineral availability, and much research is devoted to further improve these by e.g. molecular enzyme engineering. Wyss et al. (1999) Biophysical characterization of fungal phytases (myo-inositol hexakisphosphate phosphohydrolases): mulecular size, glycosylation pattern, and engineering of proteolytic resistance. Appl. Environ. Microbiol. 65. 359-366. For humans, however, no corresponding approved (food grade) enzyme is available. One way to improve the mineral state in vulnerable human populations may be to explore yeasts, that with their GRAS- (generally regarded as safe) status are preferable microorganisms in human foods. The natural yeast phytase activity during for instance bread leavening, however, seems too low to significantly influence the iron absorption when eating foods fermented by yeast, such as bread. Türk et al. (1996) Reduction in the levels of phytate during wholemeal bread making: Effect of yeast and wheat phytases. J. Cereal Science. 23. 257-264.

It is further noted that it is known from the patent literature that yeasts, such as Pichia rhodanensis (JP2000050864). Arxula adeninivorans (JP2000050863). Candida boidinii (EP 0 931 837). Saccharomyces cerevisiae (WO2001036607) may comprise genes for expression of phytase.

Greiner et al. J. Agric. Food Chem. (2001) 49(5). 2228-2233 relates to production of stereospecific myo-inositol hexaphosphate by using baker's yeast phytase.

Nakamura. et al. Biosci. Biotechnol. Biochem (2000). 64(4). 841-844 describe dephosphorylation of inositol using baker's yeast.

Turk et al. J. Agric. Food Chem. (2000). 48(1). 100-104 describes that phosphorous is stored in plants as phytates. and discuss the negative effect on mineral bioavailability and shows the ability of two commercial strains of *Saccharomyces cerevisiae* to reduce phytate by its phytase production. Turk et al express a need for more efficient phytase to be screened for or developed, although there is only evidence that phytase produced utilizes phosphorous from inositol when there is a lack of other phosphorous present.

Moore. E., et al., I. Ind. Microbiol. 14(5):396-402 (1995, May) discuss molecular cloning, expression and evaluation of phosphohydrolases for phytate degrading activity whereby four acid phosphatase genes, pho3, pho5, and pho11 from *Saccharomyces cerevisiae,* and a phosphate repressible acid phosphatase from *Aspergillus niger* were cloned by PCR and inserted into a *A*. *oryzae,* AO7. At culturing the *A. oryzae* thus transformed a two to six fold increase of phytase activity was monitored. The enzymes so produced were included into poultry diets. An available phosphorous increase was obtained. Thus Moore et al propose transformation of phosphohydrolase expressing genes from *Saccharomyces cerevisiae* to an *Aspergillus* specie in combination with an *A*. *niger* gene. There is no proposal to overexpress such phosphohydrolase producing genes in Saccharomyces cerevisiae, although the genes were picked from *S*. *cerevisiae.*

Ogawa. N., et al. Mol. Biol. Cell. 11(12):4309-21. (2000, Dec) discuss new components of a system for phosphate accumulation and polyphosphate metabolism in *Saccharomyces cerevisiae* revealed by genomic expression analysis, whereby it is stated that the PHO regulatory pathway is involved in the acquisition of phosphate in the yeast. When extracellular phosphate is low several genes are transcriptionally induced by the pathway. including pho4. pho80. pho85. There is no indication or proposal of overexpressing any of the pho family genes or deleting any of these to facilitate phytase activity on inositol under all conditions.

The PHO gene family has been extensively studied in *Saccharomyces cerevisiae.* Yoshida et al. (1989) Function of the PHO regulatory genes for repressible acid phosphatase synthesis in Saccharomyces cerevisiae. Mol. Gen. Genet. 217, 40-46; Ogawa et al. (2000) New components of a system for phosphate accumulation and polyphosphate metabolism in Saccharomyces cerevisiae revealed by genomic expression analysis. Mol. Biol. Cell. 11. 4309-4321, however, not in the context of IP6 as the substrate. The secretory acid phosphatases encoded by the structural genes *PH03, PH05, PH010* and *PH011* all seem to be aimed for hydrolyzing extracellular organic phosphorous compounds allowing the yeast to grow in the absence of inorganic phosphate. All except *PH03* are repressed by inorganic phosphate, whereas *PH03* is repressed by thiamine present in the external environment, Praekelt et al. (1994) Regulation of TH14 (MOLI), a thiamine-biosynthetic gene of Saccharomyces cerevisiae. Yeast. 10. 481-490. *PH03* is therefore suggested to primarily hydrolyze phosphate from thiamine phosphate or thiamine pyro-phosphate: Nosakaet al. (1989) A possible role for acid phosphatase with thiamin-binding activity encoded by PHO3 in yeast. FEMS Microbiol. Lett. 51. 55-59; Nosaka (1990) High affinity of acid phosphatase encoded by PHO3 gene in Saccharomyces cerevisiae for thiamin phosphates. Biochim. Biophys, Acta. 1037, 147-154

*PH05 is* often described as responsible for the major fraction of secretory acid phosphatase activity whereas *PH010* and *PH011* encode a minor fraction of secretory phosphatase. Rogers. D.T., Lemire. I.M. and Bostian, K.A. (1982) Acid phosphatase polypeptides in Saccharomyces cerevisiae are encoded by a differentially regulated multigene family. Proceedings of the National Academy of Sciences of the United States of America. 79, 2157-2161: Lemire et al. (1985) Regulation of repressible acid phosphatase gene transcription in Saccharomyces cerevisiae, Mol. Cell Biol. 5, 2131-2141, that may be lowly and constitutively expressed. In addition to the enzymes, several components within the PHO system are regulatory proteins, such as Pho4p and Pho2p which are transcriptional activators for *PH05*, *PH010 and PH011,* Vogel et al. (1989) The two positively acting regulatory proteins Pho2p and Pho4p physically interact with PHO5 upstream activation regions. Mol. Cell. Biol. 9. 2050-2057

A single study approaches the question of level of phytase activity derived from specific *S*. *cerevisiae* phosphatase encoding genes. Moore et al. (1995) Molecular cloning, expression and evaluation of phosphohydrolases for phytate-degrading activity. J. Ind. Microbiol. 14, 396-402. In that work, however, the yeast genes were all expressed in *Aspergillus* leading to uncertainty in factors such as copy number, levels of glycosylation and secretion as well as to what extent the genes contribute to phytase activity in *S*. *cerevisiae.* The opposite approach was taken by Han et al. Han et al. (1999) Expression of an Aspergillus niger phytase gene (phyA) in Saccharomyces cerevisiae. Appl. Environ. Microbiol. 65, 1915-1918, who instead expressed the *Aspergillus niger* phytase gene (*phyA*) in *S*. *cerevisiae,* to explore an alternative expression system for a well-known phytase.

### SUMMARY OF INVENTION

Broadly, the invention includes a process for preparing a *Saccharomyces cerevisiae* yeast strain having improved phytase activity comprising modifying the strain by inserting one or more of the genes *PHO5*, *PHO10, PHO11,* to overexpress said genes and by deleting one or more of the regulatory repressor genes *PHO80* and *PHO85.* whereby the insertion is either chromosomal or by transforming the strain using a plasmid containing the gene/s.

In a further preferred embodiment of the invention the modified *Saccharomyces cerevisiae* is a strain, wherein the gene *PHO80* has been deleted to form a mutant *pho80*Δ*.*

In another preferred embodiment of the invention the modified *Saccharomyces cerevisiae* is a strain, wherein the gene *PHO85* has been deleted to form a mutant *pho85*Δ*.*

In another preferred embodiment of the invention the modified *Saccharomyces cerevisiae* exhibits increased phytase activity, the strain having been deposited under DSMZ accession number DSM 14929.

In another preferred embodiment of the invention the modified *Saccharomyces cerevisiae* is a strain, wherein PHO5, PHO10 and PHO11 are overexpressed and PHO80 is deleted.

In another preferred embodiment of the invention the modified *Saccharomyces cerevisiae* is a strain wherein the strain contains a chromosomal integration of said gene/s or is transformed by a plasmid carrying said gene/s.

In yet another aspect of the invention, the invention provides a method for the preparation of phytase wherein one or more of the strains identified above are grown in a medium under conditions optimized for phytase expression whereupon lower inositol phosphates are produced as a result of yeast phytase activity on added inositol hexaphosphate.

In yet another aspect of the invention, the invention provides a method for preparing specific isomers of inositol phosphates and myo-inositiol by using one or more of the strains of the invention in a phytate and/or phytic acid containing growth medium.

I(1.2.4.5.6)P5, I(1.2.5.6)P4, I(1.2.6)P3, I(1.2.)P2, I(2)P1 and or myo-inositiol may be produced and isolated.

Besides fermented products for human use the present *Saccharomyces cerevisiae* strains can be used for the production of animal feedstuffs to increase mineral uptake, and to reduce the phosphate amounts in faeces. Phosphorous in the form of IP6 can not be resorbed in the intestines and thus animal feedstuffs are enriched using inorganic phosphates. IP6 together with excess of phosphates follow the faeces, which leads to high amounts/concentrations of phosphorous in the manure. The strains of the invention may also be used in a yeast fermentation to degrade phytate during a fermentation process.

Besides the foodstuff and feedstuff applications the enzyme phytase can be used for the manufacture of specific inositol phosphates for pharmacological use. One example is 1,2,61P3, which may stimulate the release of insuline, to reduce thrombocyte aggregation and to have anti-inflammatory properties. Such inositol phosphates may be developed into pharmaceuticals.

The enzyme phytase can also be added to foodstuffs and feedstuffs to obtain the effects specified above. Thus a further aspect of the present invention is to grow the modified *Saccharomyces cerevisiae* and to isolate the phytase enzyme produced. Such isolation is readily carried out as the phytase is present extracellularly. Primarily the addition of phytase is made to improve availability of iron and zinc to the body.

The invention also includes a modified strain of Saccharomyces cereviaisae exhibiting increased phytase activity, the strains being designated as YM-p5 and YD80.

### BRIEF DESCRIPTION OF THE DRAWINGS

FIG. 1 is a graph depicting the growth of *S*. *cerevisiae* SKQ2n (squares) and extracellular concentration of inositol hexaphosphale (IP6: triangles), measured by HPIC. as a function of time:
FIG.2 is a graph depicting the chromatographic profiles (HPIC) of chemical acid hydrolysate of sodium phytate (first line), and supernatant from *S*. *cerevisiae* cultures (second and third lines) after 15 h of growth:
FIGS. 3A and 3B are graphs depicting the growth (squares) of *S. cerevisiae* parent strain YS18 (open symbols) and *S. cerevisiae* YMR4 *pho5*Δ*pho3*Δ double deletion mutant (filed symbols) and extracellular concentration of IP6 (circles) as determined by HPlC:
FIG. 4 is a graph depicting the concentration of inositol hexaphosphate (IP6) in yeast culture supernatant as a function of growth in CBS medium with IP6 as the sole phosphorous source:
FIG. 5 is a graph depicting the concentration of inositol hexaphosphate (IP6) in yeast culture supernatant as a function of growth in complex YPD medium with IP6 as the added phosphorous source.
FIG. 6 is a vector construct consisting of plasmid pYX212 containing the insert *PHO5* used for transformation of YS18 and YMR4:
FIGS. 7A and 7B are graphs depicting the extracellular concentration of IP6 as a function of growth time of *S*. *cerevisiae* YMR4 (A) and YS18 (B), with and without the *PHO5* containing plasmid pYX212:
FIG. 8 is a graph depicting a direct comparison of phytate degrading capacity between *PHO5* overexpressing strains, and *pho80*Δ and *pho85*Δ deletions mutants:
FIG. 9 is a graph depicting phytate degrading capacity of *pho2*Δ deletion mutant:
FIG. 10 is a graph depicting the extracellular concentration of para-nitrophenyl phosphate (pNPP. squares) and IP6 (triangles) as a function of growth of *S*. *cerevisiae* SKQ2n in CBS medium with 2% glucose as carbon and energy source:
FIG. 11. is a graph depicting the content of inositol hexaphosphate (IP6. triangles) and inorganic phosphate (Pᵢ: squares), expressed as µmol per gram wet dough, as a function of leavening time in two types of wheat based dough: and
FIG. 12 is a graph depicting phytate degradation capacity of the *pho80*Δ deletion mutant SD80 (YS18 without *PH080*) compared with *S*. *cerevisiae* YS18 in YPD supplemented with 0.25 mM IP6 and 30 mM Pi.

### DETAILED DESCRIPTION OF INVENTION

The invention will now be described in reference to the following non-timiting examples.

### Materials and methods

### Organisms

*Saccharomyces cerevisiae* SKQ2n. Bataille et al. (1987) Identification of polypeptides of the carbon metabolism machinery on the two-dimensional map of Saccharomyces cerevisiae. Location of 23 additional polypeptides. Yeast. 3, 11-21., *S*. *cerevisiae* YS18 (MATα: *his3*-11, 3-15: *leu2*-3, 2-112: *ura3*Δ*5*; canR) and *S*. *cerevisiae* YMR4 were used in most experiments as indicated. YS18 and YMR4 were previously constructed by Riederer and I-linnen: Riederer et al. (1991) Removal of N-glycosylation sites of the yeast acid phosphatase severely affects protein folding. J. Bacteriol. 173.3539-3546, YS18 and YMR4 were in the present work both transformed with a *PHO5* containing plasmid (see below) and the resulting transformant strains were designated YS-p5 and YM-p5. respectively, Strain YS-p5 was deposited with DSMZ - Deutsche Sammlung von Mikroorganismen und Zellkulturen GmbH, Mascheroder Weg lb, 38124 Braunschweig. Germany on April 18, 2002 and was assigned accession number DSM-No. 14929.

YS18 and YMR4 were further deleted for *PHO10* yielding strains named YS10 and YM10, respectively, YS18 was also used for construction of the *pho80*Δ strain labelled YD80. In addition, *pho80*Δ. *pho85*Δ and *pho2*Δ mutant strains were retrieved from the German EUROSCARF collection. *pho80* (YOL001w), *pho85* (YPL031c) and *pho2* (YDL106c) strains originates from the laboratory BY474l strain (*MATa*: *his*3Δ*1*. *leu*2Δ*0*, *met 15*Δ*0*. *ura3*Δ*0*). The *PHO80*, *PHO85* and *PHO2* genes were deleted with *kan::MX4* disruption cassette.

Yeast cultures were maintained on YPD-plates (yeast extract 10g. peptone 20 g. glucose 20 g and agar 20 g in one liter of water) and stored long-term in glycerol at -70°C. *Escherichia coli* DH5α was used for propagating the plasmid containing the inserted *PHO5* (see below). The yeast strains and their expression profiles of relevant genes are listed in Table 1.

**Table 1. Strains of Saccharomyces cerevisiae used in this study, their relevant genotype and expression profiles of secretory phosphatases in repressing and non-repressing media.**

| **Strain** | **Relevant genotype** | **Secretory phosphates expressed in IP₆ medium** | **^{a}Secretory phosphates expressed in IP₆ + Pᵢ medium** | **Reference for strain** |
|---|---|---|---|---|
| SKQ2n | all *PHO* genes intact | Pho5p. Pho10p. Pho 11p | none | Bataille et al. (1987) |
| YSI8 | all *PHO* genes intact. Parent strain for YMR4 | Pho5p. Pho10p. Pho11p | none | Riederer ei et al. (1991) |
| YMR4 | *pho5.3::ura*Δ*1* | Pho10p. Pho11p | none | Riederet et al. (1991) |
| YM-p5 | *pho5.3.: uraΔ1* pYX-PHO5 | Pho5p^{b}.Pho10p. Pho11p | Pho5p^{b} | This work |
| YS-p5 | pYX-PHO5 | Pho5p^{b}. Pho5p. Pho10p. Pho11p | Pho5p^{b} | This work |
| YM10 | *pho5.3::ura3*Δ*1 pho10::his3*Δ | Pho11p | none | This work |
| YS10 | *pho10::his3*Δ | Pho5p.Pho11p | none | This work |
| BY4741 | All *PHO* genes intact | Pho5p. Pho 10p. Pho11p | none | EUROSCARF |
| YOL001w | *pho80*Δ | Pho5p. Pho10p. Pho11p | Pho5p. Pho10p. Pho11p | EUROSCARF |
| YPL031c | *pho85*Δ | Pho5p. Pho10p. Pho11p | Pho5p. Pho10p. Pho11p | EUROSCARF |
| YDL106c | *pho2*Δ | none | none | EUROSCARF |
| YD80 | *pho80*Δ..*hph* | Pho5p. Pho10p. Pho11p | Pho5p. Pho10p. Pho11p | This work |

| | | | | |
|---|---|---|---|---|
| a) A small constitutive expression may occur b) On the plasmid pYX212 under the control of TP11 promoter | | | | |

### Growth media and culture conditions

The growth medium used in most experiments was a modified version or the defined yeast minimal medium developed by CBS (Centralbueau voor Schimmelcultures. Delft the Netherlands) which has been described previously. Albers et al. (1996) *Influence of the nitrogen source on Saccharomyces cerevisiae anaerobic growth and product formation.* Appl. Environ, Microbiol, 62. 3187-3195 using ammonium sulfate (7.5 mg/ml) as nitrogen source. As phosphorus source, either IP6 in the form of sodium phytate (C₆H₆(OPO₃Na₂)₆: 0.5 or 0.25 mg/ml), inorganic phosphate (KH₂PO₄: 3.5 mg/ml), para-nitropheny) phosphate (pNPP: to a final concentration of 2.25 or 0.25 mM), or combinations or these were used. Depending on the phosphorous source used the media were designated CBSIP6, CBSP₁, CBSIP6+P₁ and CBSpNPP, CBSIP6 and CBSpNPP were supplemented with KCl (3 mg/ml) to compensate for the potassium present in the inorganic P-source (KH₂PO₄), but absent in the organic P-sources. In all experiments glucose (either 2% (w/v) or 1% (w/v) as indicated) was used as carbon and energy source. When appropriate, histidine, leucine and uracil were supplied at 120 mg/). Most experiments were also performed in YPD media (per liter: yeast extract, 10 g: peptone 20 g and glucose 20 g) supplemented with the appropriate P-sources as indicated in results and figure legends. Succinic acid/NaOH. p 5,3. was used as buffer in the CBS based media. Medium components were autoclaved, with the exception for IP6. pNPP, vitamins, growth factors and FeCl₂, which were sterilized by filtration through a 0.2 µm filter. Experimental cultures containing 100 ml in 250 ml E-flasks were inoculated with primary cultures grown for approximately 20h in medium of the same composition as the respective experiment culture. The inoculation level was set to OD₆₁₀ = 0.2. The experimental cultures were grown at 30°C in a rotary shaker set to 210 rpm. The growth was monitored as optical density at 610 nm (OD₆₁₀) using a spectrophotometer (Hitachi, model U-100). Experiments aimed at *PHO3* expression were performed in CBS medium as described above with the exception of excluding thiamine (to avoid repression of the thiamine phosphatase *PHO3*), with and without addition of thiamine monophosphate.

### Construction of PHO5 overexpressing strain.

Chemicals and enzymes for the procedures were purchased from New England Biolabs Inc., USA. and the PCR primers used for amplification of *PHO5* were purchased from Life Technologies AB, Täby, Sweden. Extraction of genomic DNA from *S*. *cerevisiae* YS18 was carried out according to standard procedures. The nucleotide sequences of the primers are shown in upper case letters and the added restriction enzyme sites are shown in lower case letters.

### Primers for overexpression of PHO5

- *Forward primer:*
   5': CGGAATTCATGTTTAAATCTGTTGTTTATTC, appended as SEQ ID NO: 1
- *Backwards primer:*
   3': CGCTCGAGCTATTGTCTCAATAGACTGGC, appended as SEQ ID NO: 2 Underlined sequences are EcoRI and Aval (yields end compatible to Xhol used to cut the pYX212 plasmid) restriction sites, respectively. The remaining sequences complement the beginning (from ATG) and end of *PHO5*.

The PCR reaction was performed in 100 µl reaction mixtures, using VENT polymerase and was carried out according to standard procedures.

The PCR produced *PHO5* was purified with a PCR purification kit (Qiagen, Cat. No. 28104. Merck Eurolab AB, Sweden) according to the manufacturer's protocol and 50 µl was cut with Aval and EcoRI in EcoRI buffer for 12h at 37°C. The cut PCR product was loaded on a preparative agarose gel and run together with a DNA ladder in TAE buffer. The appropriate band was cut out and purified using DNA get extraction kit (Qiagen, Cat. No. 28704). A 20 µl ligation mixture was prepared by mixing 16 µl purified insert (*PHO5*). 2 µl T4 DNA ligase buffer (10X) 1 µl ligase and 2 µl vector DNA (pYX212). The plasmid pYX212 containing the selection markers Amp^{R} for *E. coli* and *URA3* for *S. cerevisiae* had previously been cut with EcoRI and Xhol (yields ends compatible with Aval). Two µl of the DNA construct was added to 40 µl of cold competent *E. coli* DH5α and transformed by electroporation using a Gene Pulser II (Biorad) set to the 25 µl capacitator, 2.5 kV and the pulse controller to 200 Ω. Cells were plated on NB plates containing 100 µg/ml ampicillin. Clones were cultivated over night in liquid NB medium and plasmids were prepared according to standard procedures.

Sequence of the plasmid pYX212 with insert *PHO5:*

GAATTCATGTTTAAATCTGTTGTTTATTCAATTTTAGCCGCTTCTTTGGCC
AATGCAGGTACCATTCCCTTAGGCAAACTAGCCGATGTCGACAAGATTGG
TACCCAAAAAGATATCTTCCCATTTTTGGGTGGTGCCGGACCATACTACT
CTTTCCCTGGCGACTATGGTATTTCTCGTGATTTGCCTGAAGGTTGTGAA
ATGAAGCAACTGCAAATGGTTGGTAGACATGGTGAAAGATACCCTACTGT
CAGTCTGGCTAAGACTATCAAGAGTACATGGTATAAGTTGAGCAATTACA
CTCGTCAATTCAACGGCTCATTGTCATTCTTGAACGATGATTACGAGTTT
TTCATCCGTGATGACGATGATTTGGAAATGGAAACCACTTTTGCCAACTC
GGACGATGTTTTGAACCCATACACTGGTGAAATGAACGCCAAGAGACAT
GCTCGTGACTTCTTGGCTCAATACGGTTACATGGTCGAAAACCAAACCAG
TTTCGCCGTTTTTACCTCTAATTCTAAGAGATGTCATGACACTGCTCAATA
TTTCATTGATGGTTTAGGTGACCAATTCAACATCACCTTGCAGACTGTCA
GTGAAGCTGAATCCGCTGGTGCCAACACTTTGAGTGCTTGTAACTCATGT
CCTGCTTGGGACTACGATGCCAATGATGACATTGTAAATGAATACGACAC
AACCTACTTGGATGACATTGCCAAGAGATTGAACAAGGAAAACAAGGGT
TTGAACTTGACCTCAACTGACGCTAGTACTTTATTCTCGTGGTGTGCATT
TGAAGTGAACGCTAAAGGTTACAGTGATGTCTGTGATATTTTCACCAAGG
ATGAATTAGTCCATTACTCCTACTACCAAGACTTGCACACTTATTACCAT
GAGGGTCCAGGTTACGACATTATCAAGTCTGTCGGTTCCAACTTGTTCAA
TGCCTCAGTCAAATTATTAAAGCAAAGTGAGATTCAAGACCAAAAGGTTT
GGTTGAGTTTTACCCACGATACCGATATCCTAAACTTTTTGACCACCGCT
GGTATAATTGACGACAAAAACAACTTAACTGCCGAATACGTTCCATTCAT
GGGCAACACTTTCCACAGATCCTGGTACGTTCCTCAAGGTGCTCGTGTCT
ACACCGAAAAATTCCAATGTTCTAACGACACCTACGTCAGATACGTCATT
AACGATGCTGTTGTTCCAATTGAAACCTGTTCCACTGGTCCAGGGTTCTC
TTGTGAAATCAATGACTTCTACGACTATGCTGAAAAGAGAGTAGCCGGTA
CTGACTTCCTAAAGGTCTGTAACGTCAGCAGCGTCAGTAACTCTACTGAA
TTGACCTTCTACTGGGACTGGAACACTACTCATTACAACGCCAGTCTATT
GAGACAATAGCCCGGGTATCCGTATGATGTGCCTGACTACGCATGATATCTC
GAGCTCAGCTAGCTAACTGAATAAGGAACAATGAACGTTTTTCCTTTCTCTTG
TTCCTAGTATTAATGACTGACCGATACATCCCTTTTTTTTTTTGTCTTTGTCTAG
CTCCAGCTTTTGTTCCCTTTAGTGAGGGTTAATTCAATTCACTGGCCGTCGTTT
TACAACGTCGTGACTGGGAAAACCCTGGCGTTACCCAACTTAATCGCCTTGCA
GCACATCCCCCTTTCGCCAGCTGGCGTAATAGCGAAGAGGCCCGCACCGATC
GCCCTTCCCAACAGTTGCGCAGCCTGAATGGCGAATGGCGCGACGCGCCCTG
TAGCGGCGCATTAAGCGCGGCGGGTGTGGTGGTTACGCGCAGCGTGACCGCT
ACACTTGCCAGCGCCCTAGCGCCCGCTCCTTTCGCTTTCTTCCCTTCCTTTCTC
GCCACGTTCGCCGGCTTTCCCCGTCAAGCTCTAAATCGGGGGCTCCCTTTAGG
GTTCCGATTTAGTGGTTTACGGCACCTCGACCCCAAAAAACTTGATTAGGGTG
ATGGTTCACGTAGTGGGCCATCGCCCTGATAGACGGTTTTTCGCCCTTTGACG
TTGGAGTCCACGTTCTTTAATAGTGGACTCTTGTTCCAAACTGGAACAACACT
CAACCCTATCTCGGTCTATTCTTTTGATTTATAAGGGATTTTGCCGATTTCGGC
CTATTGGTTAAAAAATGAGCTGATTTAACAAAAATTTAACGCGAATTTTAACA
AAATATTAACGTTTACAATTTCCTGATGCGGTATTTTCTCCTTACGCATCTGTG
CGGTATTTCACACCGCATAGGGTAATAACTGATATAATTAAATTGAAGCTCTA
ATTTGTGAGTTTAGTATACATGCATTTACTTATAATACAGTTTTTTAGTTTTGC
TGGCCGCATCTTCTCAAATATGCTTCCCAGCCTGCTTTTCTGTAACGTTCACCC
TGTACCTTAGCATCCCTTCCCTTTGCAAATAGTCCTCTTCCAACAATAATAATG

TCAGATCCTGTAGAGACCACATCATCCACGGTTCTATACTGTTGACCCAATGC
GTCTCCCTTGTCATCTAAACCCACACCGGGTGTCATAATCAACCAATCGTAAC
CTTCATCTCTTCCACCCATGTCTCTTTGAGCAATAAAGCCGATAACAAAATCT
TTGTCGCTCTTCGCAATGTCAACAGTACCCTTAGTATATTCTCCAGTAGATAG
GGAGCCCTTGCATGACAATTCTGCTAACATCAAAAGGCCTCTAGGTTCCTTTG
TTACTTCTTCTGCCGCCTGCTTCAAACCGCTAACAATACCTGGCCCAGCACA
CCGTGTGCATTCGTAATGTCTGCCCATTCTGCTATTCTGTATACACCCGCAGA
GTACTGCAATTTGACTGTATTACCAATGTCAGCAAATTTTCTGTCTTCGAAGA
GTAAAAAATTGTACTTGGCGGATAATGCCTTTAGCGGCTTAACTGTGCCCTCC
ATCGAAAAATCAGTCAATATATCCACATGTGTTTTTAGTAAACAAATTTTGGG
ACCTAATGCTTCAACTAACTCCAGTAATTCCTTGGTGGTACGAACATCCAATG
AAGCACACAAGTTTGTTTGCTTTTCGTGCATGATATTAAATAGCTTGGCAGCA
ACAGGACTAGGATGAGTAGCAGCACGTTCCTTATATGTAGCTTTCGACATGAT
TTATCTTCGTTTCCTGCAGGTTTTTGTTCTGTGCAGTTGGGTTAAGAATACTGG
GCAATTTCATGTTTCTTCAACACTACATATGCGTATATATACCAATCTAAGTCT
GTGCTCCTTCCTTCGTTCTTCCTTCTGTTCGGAGATTACCGAATCAAAAAAATT
TCAAAGAAACCGAAATCAAAAAAAAGAATAAAAAAAAAATGATGAATTGAA
TTGAAAAGCTGTGGTATGGTGCACTCTCAGTACAATCTGCTCTGATGCCGCAT
AGTTAAGCCAGCCCCGACACCCGCCAACACCCGCTGACGCGCCCTGACGGGC
TTGTCTGCTCCCGGCATCCGCTTACAGACAAGCTGTGACCGTCTCCGGGAGCT
GCATGTGTCAGAGGTTTTCACCGTCATCACCGAAACGCGCGAGACGAAAGGG
CCTCGTGATACGCCTATTTTTATAGGTTAATGTCATGATAATAATGGTTTCTTA
GACGTGCGGCCGCTCTAGAACTAGTGGATCAATTCCACGGACTATAGACTAT
ACTAGTATACTCCGTCTACTGTACGATACACTTCCGCTCAGGTCCTTGTCCTTT
AACGAGGCCTTACCACTCTTTTGTTACTCTATTGATCCAGCTCAGCAAAGGCA
GTGTGATCTAAGATTCTATCTTCGCGATGTAGTAAAACTAGCTAGACCGAGAA
AGAGACTAGAAATGCAAAAGGCACTTCTACAATGGCTGCCATCATTATTATCC
GATGTGACGCTGCAGCTTCTCAATGATATTCGAATACGCTTTGAGGAGATACA
GCCTAATATCCGACAAACTGTTTTACAGATTTACGATCGTACTTGTTACCCAT
CATTGAATTTTGAACATCCGAACCTGGGAGTTTTCCCTGAAACAGATAGTATA
TTTGAACCTGTATAATAATATATAGTCTAGCGCTTTACGGAAGACAATGTATG
TATTTCGGTTCCTGGAGAAACTATTGCATCTATTGCATAGGTAATCTTGCACG
TCGCATCCCCGGTTCATTTTCTGCGTTTCCATCTTGCACTTCAATAGCATATCT
TTGTTAACGAAGCATCTGTGCTTCATTTTGTAGAACAAAAATGCAACGCGAGA
GCGCTAATTTTTCAAACAAAGAATCTGAGCTGCATTTTTACAGAACAGAAATG
CAACGCGAAAGCGCTATTTTACCAACGAAGAATCTGTGCTTCATTTTTGTAAA
ACAAAAATGCAACGCGAGAGCGCTAATTTTTCAAACAAAGAATCTGAGCTGC
ATTTTTACAGAACAGAAATGCAACGCGAGAGCGCTATTTTACCAACAAAGAA
TCTATACTTCTTTTTTGTTCTACAAAAATGCATCCCGAGAGCGCTATTTTTCTA
ACAAAGCATCTTAGATTACTTTTTTTCTCCTTTGTGCGCTCTATAATGCAGTCT
CTTGATAACTTTTTGCACTGTAGGTCCGTTAAGGTTAGAAGAAGGCTACTTTG
GTGTCTATTTTCTCTTCCATAAAAAAAGCCTGACTCCACTTCCCGCGTTTACTG
ATTACTAGCGAAGCTGCGGGTGCATTTTTTCAAGATAAAGGCATCCCCGATTA
TATTCTATACCGATGTGGATTGCGCATACTTTGTGAACAGAAAGTGATAGCGT
TGATGATTCTTCATTGGTCAGAAAATTATGAACGGTTTCTTCTATTTTGTCTCT
ATATACTACGTATAGGAAATGTTTACATTTTCGTATTGTTTTCGATTCACTCTA
TGAATAGTTCTTACTACAATTTTTTTGTCTAAAGAGTAATACTAGAGATAAAC
ATAAAAAATGTAGAGGTCGAGTTTAGATGCAAGTTCAAGGAGCGAAAGGTGG
ATGGGTAGGTTATATAGGGATATAGCACAGAGATATATAGCAAAGAGATACT
TTTGAGCAATGTTTGTGGAAGCGGTATTCGCAATATTTTAGTAGCTCGTTACA

GTCCGGTGCGTTTTTGGTTTTTTGAAAGTGCGTCTTCAGAGCGCTTTTGGTTTT
CAAAAGCGCTCTGAAGTTCCTATACTTTCTAGAGAATAGGAACTTCGGAATAG
GAACTTCAAAGCGTTTCCGAAAACGAGCGCTTCCGAAAATGCAACGCGAGCT
GCGCACATACAGCTCACTGTTCACGTCGCACCTATATCTGCGTGTTGCCTGTA
TATATATATACATGAGAAGAACGGCATAGTGCGTGTTTATGCTTAAATGCGTA
CTTATATGCGTCTATTTATGTAGGATGAAAGGTAGTCTAGTACCTCCTGTGAT
ATTATCCCATTCCATGCGGGGTATCGTATGCTTCCTTCAGCACTACCCTTTAGC
TGTTCTATATGCTGCCACTCCTCAATTGGATTAGTCTCATCCTTCAATGCTATC
ATTTCCTTTGATATTGGATCATATGCATAGTACCGAGAAACTAGTGCGAAGTA
GTGATCAGGTATTGCTGTTATCTGATGAGTATACGTTGTCCTGGCCACGGCAG
AAGCACGCTTATCGCTCCAATTTCCCACAACATTAGTCAACTCCGTTAGGCCC
TTCATTGAAAGAAATGAGGTCATCAAATGTCTTCCAATGTGAGATTTTGGGCC
ATTTTTTATAGCAAAGATTGAATAAGGCGCATTTTTCTTCAAAGCTGCGGCCG
CACTCTCACTAGTACGTCAGGTGGCACTTTTCGGGGAAATGTGCGCGGAACCC
CTATTTGTTTATTTTTCTAAATACATTCAAATATGTATCCGCTCATGAGACAAT
AACCGTGATAAATGCTTCAATAATATTGAAAAAGGAAGAGTATGAGTATTCA
ACATTTCCGTGTCGCCCTTATTCCCTTTTTTGCGGCATTTTGCCTTCCTGTTTTT
GCTCACCCAGAAACGCTGGTGAAAGTAAAAGATGCTGAAGATCAGTTGGGTG
CACGAGTGGGTTACATCGAACTGGATCTCAACAGCGGTAAGATCCTTGAGAG
TTTTCGCCCCGAAGAACGTTTTCCAATGATGAGCACTTTTAAAGTTCTGCTAT
GTGGCGCGGTATTATCCCGTATTGACGCCGGGCAAGAGCAACTCGCTCGCCG
CATACACTATTCTCAGAATGACTTGGTTGAGTACTCACCAGTCACAGAAAAGC
ATCTTACGGATGGCATGACAGTAAGAGAATTATGCAGTGCTGCCATAACCAT
GAGTGATAACACTGCGGCCAACTTACTTCTGACAACGATCGGAGGACCGAAG
GAGCTAACCGCTTTTTTGGACAACATGGGGGATCATGTAACTCGCCTTGATCG
TTGGGAACCGGAGCTGAATGAAGCCATACCAAACGACGAGCGTGACACCACG
ATGCCTGTAGCAATGGCAACAACGTTGCGCAAACTATTAACTGGCGAACTAC
TTACTCTAGCTTCCCGGCAACAATTAATAGACTGGATGGAGGCGGATAAAGTT
GCAGGACCACTTCTGCGCTCGGCCCTTCCGGCTGGCTGGTTTATTGCTGATAA
ATCTGGAGCCGGTGAGCGTGGGTCTCGCGGTATCATTGCAGCACTGGGGCCA
GATGGTAAGCCCTCCCGTATCGTAGTTATCTACACGACGGGGAGTCAGGCAA
CTATGGATGAACGAAATAGACAGATCGCTGAGATAGGTGCCTCACTGATTAA
GCATTGGTAACTGTCAGACCAAGTTTACTCATATATACTTTAGATTGATTTAA
AACTTCATTTTTAATTTAAAAGGATCTAGGTGAAGATCCTTTTTGATAATCTCA
TGACCAAAATCCCTTAACGTGAGTTTTCGTTCCACTGAGCGTCAGACCCCGTA
GAAAAGATCAAAGGATCTTCTTGAGATCCTTTTTTTCTGCGCGTAATCTGCTG
CTTGCAAACAAAAAAACCACCGCTACCAGCGGTGGTTTGTTTGCCGGATCAA
GAGCTACCAACTCTTTTTCCGAAGGTAACTGGCTTCAGCAGAGCGCAGATACC
AAATACTGTCCTTCTAGTGTAGCCGTAGTTAGGCCACCACTTCAAGAACTCTG
TAGCACCGCCTACATACCTCGCTCTGCTAATCCTGTTACCAGTGGCTGCTGCC
AGTGGCGATAAGTCGTGTCTTACCGGGTTGGACTCAAGACGATAGTTACCGG
ATAAGGCGCAGCGGTCGGGCTGAACGGGGGGTTCGTGCACACAGCCCAGCTT
GGAGCGAACGACCTACACCGAACTGAGATACCTACAGCGTGAGCTATGAGAA
AGCGCCACGCTTCCCGAAGGGAGAAAGGCGGACAGGTATCCGGTAAGCGGC
AGGGTCGGAACAGGAGAGCGCACGAGGGAGCTTCCAGGGGGAAACGCCTGG
TATCTTTATAGTCCTGTCGGGTTTCGCCACCTCTGACTTGAGCGTCGATTTTTG
TGATGCTCGTCAGGGGGGCGGAGCCTATGGAAAAACGCCAGCAACGCGGCCT
TTTTACGGTTCCTGGCCTTTTGCTGGCCTTTTGCTCACATGTTCTTTCCTGCGTT
ATCCCCTGATTCTGTGGATAACCGTATTACCGCCTTTGAGTGAGCTGATACCG
GTCGCCGCAGCCGAACGACCGAGCGCAGCGAGTCAGTGAGCGAGGAAGCGG

AAGAGCGCCCAATACGCAAACCGCCTCTCCCCGCGCGTTGGCCGATTCATTAA
TGCAGCTGGCACGACAGGTTTCCCGACTGGAAAGCGGGCAGTGAGCGCAACG
CAATTAATGTGAGTTACCTCACTCATTAGGCACCCCAGGCTTTACACTTTATG
CTTCCGGCTCCTATGTTGTGTGGAATTGTGAGCGGATAACAATTTCACACAGG
AAACAGCTATGACCATGATTACGCCAAGCTCGAAATACGACTCACTATAGGG
CGAATTGGGTACCGGGCCGGCCGTCGAGCTTGATGGCATCGTGGTGTCACGCT
CGTCGTTTGGTATGGCTTCATTCAGCTCCGGTTCCCAACGATCAAGGCGAGTT
ACATGATCCCCCATGTTGTGAAAAAAGCGGTTAGCTCTTCGGTCCTCCGATC
GTTGTCAGAAGTAAGTTGGCCGCAGTGTTATCACTCATGGTTATGGCAGGAAC
TGCATAATTCTCTTACTGTCATGCCATCCGTAAGATGCTTTTCTGTGACTGGTG
TACTCAACCAAGTCATTCTGAGAATAGTGTATGCGGCGACCGAGTTGCTCTTG
CCCGGCGTCAACACGGGATAATACCGCGCCACATAGCAGAACTTTAAAAGTG
CTCATCATTGGAAAACGTTCTTCGGGGCGAAAACTCTCAAGGATCTTACCGCT
GTTGAGATCCAGTTCGATGTAACCCACTCGTGCACCCAACTGATCTTCAGCAT
CTTTTACTTTCACCAGCGTTTCTGGGTGAGCAAAAACAGGAAGGCAAAATGCC
GCAAAAAAGGGAATAAGGGCGACACGGAAATGTTGAATACTCATACTCTTCC
TTTTTCAATATTATTGAAGCATTTATCAGGGTTATTGTCTCATGAGCGATACAT
ATTTGAATGTATTTAGAAAAATAAACAAATAGGGGTTCCGCGCACATTTCCCC
GAAAAGTGCCACCTGACGTCTAAGAAACCATTATTATCATGACATTAACCTAT
AAAAATAGGCGTATCACGAGGCCCTTTCGTCTTCAAGAATTGGGGATCTACGT
ATGGTCATTCTTCTTCAGATTCCCTCATGGAGAAGTGCGGCAGATGTATATGA
CAGAGTCGCCAGTTTCCAAGAGACTTTATTCAGGCACTTCCATGATAGGCAAG
AGAGAAGACCCAGAGATGTTGTTGTCCTAGTTACACATGGTATTTATTCCAGA
GTATTCCTGATGAAATGGTTTAGATGGACATACGAAGAGTTTGAATCGTTTAC
CAATGTTCCTAACGGGAGCGTAATGGTGATGGAACTGGACGAATCCATCAAT
AGATACGTCCTGAGGACCGTGCTACCCAAATGGACTGATTGTGAGGGAGACC
TAACTACATAGTGTTTAAAGATTACGGATATTTAACTTACTTAGAATAATGCC
ATTTTTTTGAGTTATAATAATCCTACGTTAGTGTGAGCGGGATTTAAACTGTG
AGGACCTCAATACATTCAGACACTTCTGACGGTATCACCCTACTTATTCCCTT
CGAGATTATATCTAGGAACCCATCAGGTTGGTGGAAGATTACCCGTTCTAAGA
CTTTTCAGCTTCCTCTATTGATGTTACACTCGGACACCCCTTTTCTGGCATCCA
GTTTTTAATCTTCAGTGGCATGTGAGATTCTCCGAAATTAATTAAAGCAATCA
CACAATTCTCTCGGATACCACCTCGGTTGAAACTGACAGGTGGTTTGTTACGC
ATGCTAATGCAAAGGAGCCTATATACCTTTGGCTCGGCTGCTGTAACAGGGA
ATATAAAGGGCAGCATAATTTAGGAGTTTAGTGAACTTGCAACATTTACTATT
TTCCCTTCTTACGTAAATATTTTTCTTTTTAATTCTAAATCAATCTTTTTCAATT
TTTTGTTTGTATTCTTTTCTTGCTTAAATCTATAACTACAAAAAACACATACAG
G     appended as SEQ ID NO: 3.

Underlined sequences show sites for primers
Bolded nucleotides represent the inserted *PHO5*

The presence of *PHO5* was verified by PCR using the same primers as described above as well as by cutting with EcoRV followed by gel electrophoresis, then transformed into yeast strains YS18 and YMR4 using a standard LiOAc *S. cerevisiae* transformation protocol. Yeasts containing the vector with insert were selected for on uracil negative YNB plates (DIFCO, USA) containing the appropriate amino acids. The resulting transformant strains were named YS-p5 and YM-p5, respectively.

### Construction of deletion strains

The *PHO10* deletions were generated by PCR-mediated gene replacement. Baudin et al. (1993) A simple and efficient for direct gene deletion in Saccharomyces cerevisiae. Nucleic Acids Res. 21. 3329-3330 using *HIS5* from *Saccharomyces pombe* (corresponding to *HIS3* in *Saccharomyces cerevisiae)* as the selectable marker. As template DNA the plasmid pFA6a-HIS3MX6: Wach et al. (1994) New heterologous modules for classical or PCR-based gene disruptions in Saccharomyces cerevisiae, Yeast, 10, 1793-1808 was used.

### Primers for deletion of PHO10

5':
CGATAGATTCAAGCTCAGTTTCGCCTTGGTTGTAAAGTAGGCAGCTGAAGCTT
CGTACGC-3'. appended as SEQ ID NO: 4

5`:
GGTCTATTTACTGTTTTAATAAAGTGTCGTTGTAGTGCTTGGGGCAGATGATG
TCGAGGCG-3`. appended as SEQ ID NO 5

These oligonucleotides were used as primers for construction of deletion cassettes. Underlined sequences are complementary to *HIS5* in the template, and non-underlined parts to flanking regions of *PHO10*. The *PHO10* deletions were generated by PCR-mediated gene replacement using *HIS5* from *Saccharomyces pombe* (corresponding to *HIS3* in *S*. *cerevisiae*) as the selectable marker. As template DNA the plasmid pFA6a-HIS3MX6 was used.

Sequence of the Resulting PCR product used for deletion of *PHO10* by homologous recombination: 
CGATAGATTCAAGCTCAGTTTCGCCTTGGTTGTAAAGTAGCCAGCTGAAGCTTCGT
ACGCTGCAGGTCGACGGATCCCCGGGTTAATTAAGGCGCGCCAGATCTGTTTA
 
GCTTGCCTCGTCCCCGCCGGGTCACCCGGCCAGCGACATGGAGGCCCAGAAT
ACCCTCCTTGACAGTCTTGACGTGCGCAGCTCAGGGGCATGATGTGACTGTCG
CCCGTACATTTAGCCCATACATCCCCATGTATAATCATTTGCATCCATACATTT
TGATGGCCGCACGGCGCGAAGCAAAAATTACGGCTCCTCGCTGCAGACCTGC
GAGCAGGGAAACGCTCCCCTCACAGACGCGTTGAATTGTCCCCACGCCGCGC
CCCTGTGAGAAATATAAAAGGTTAGGATTTGCCACTGAGGTTCTTCTTTCATA
TACTTCCTTTTAAATCTTGCTAGGATACAGTTCTCACATCACATCCGAACATA
AACAACCATGGGTAGGAGGGCTTTTGTAGAAAGAAATACGAACGAAACGAA
AATCAGCGTTGCCATCGCTTTGGACAAAGCTCCCTTACCTGAAGAGTCGAATT
TTATTGATGAACTTATAACTTCCAAGCATGCAAACCAAAAGGGAGAACAAGT
AATCCAAGTAGACACGGGAATTGGATTCTTGGATCACATGTATCATGCACTGG
CTAAACATGCAGGCTGGAGCTTACGACTTTACTCAAGAGGTGATTTAATCATC
GATGATCATCACACTGCAGAAGATACTGCTATTGCACTTGGTATTGCATTCAA
GCAGGCTATGGGTAACTTTGCCGGCGTTAAAAGATTTGGACATGCTTATTGTC
CACTTGACGAAGCTCTTTCTAGAAGCGTAGTTGACTTGTCGGGACGGCCCTAT
GCTGTTATCGATTTGGGATTAAAGCGTGAAAAGGTTGGGGAATTGTCCTGTGA
AATGATCCCTCACTTACTATATTCCTTTTCGGTAGCAGCTGGAATTACTTTGCA
TGTTACCTGCTTATATGGTAGTAATGACCATCATCGTGCTGAAAGCGCTTTTA
AATCTCTGGCTGTTGCCATGCGCGCGGCTACTAGTCTTACTGGAAGTTCTGAA
GTCCCAAGCACGAAGGGAGTGTTGTAAAGAGTACTGACAATAAAAAGATTCT
TGTTTTCAAGAACTTGTCATTTGTATAGTTTTTTTATATTGTAGTTGTTCTATTT
TAATCAAATGTTAGCGTGATTTATATTTTTTTTCGCCTCGACATCATCTGCCCC
AAGCACTACAACGACACTTTATTAAAACAGTAAATAGACC
 appended as SEQ ID NO: 6

### Underlined sequences represent primers (or complementary strand to primer) used Italic shows sequence corresponding to PHO10 (SGD)

The PCR reactions were performed in 100 µl using VENT polymerase, the crude PCR product was purified by cutting the appropriate band from a preparative agorase gel-electrophoresis and YMR4 and YS18 were transformed by the lithium acetate method resulting in strains named YM10 and YS10, respectively. The deletions were verified by PCR on whole yeast colonies as well as on extracted DNA.

Deletion of *PHO80* was performed by PCR-based gene disruption, mainly according to Baudin et al. (1993) A simple and efficient for direct gene deletion in Saccharomyces Cerevisiaw. Nucleic Acids Res. 21. 3329-3330. and Goldstein and McCusker, (1999) New heterotogous modules for classical or PCR-based gene disruptions in Saccharomyces cerevisiae. Yeast, 10, 1793-1808. A deletion fragment consisting of a selectable marker flanked by 5' and 3' flanking sequences of *PHO80* was constructed. At transformation, the *PHO80* ORF was exchanged for hygromycin B phosphotransferase *(hph)* by homologous recombination. The plasmid pAG32 Goldstein et al., containing the selectable marker *hph* (hygromycin B phosphotransferase) was used as template for the PCR based construction of the *PHO80* deletion fragment. For the *PHO80* region, primers were designed according to SGD (http://genome-w.ww.stanford.edu/Saccharomyces/) and for the *hph* region according to Gritz and Davis (1983) "Plasmid encoded hygromycin B resistance: the sequence of hygromycin B phosphotransferase gene and its expression in Escherichia coli and Saccharomyces cerevisiae." Gene. 25. 178-188..

### Deletion of P^{H}080

- *Forward primer:* 
5': CAGCGTATATTGGCTTTCCTTTAATCTAATGCCCCAAGCCCACAT
ACGATTTAGGTGACAC -3'    APPENDED AS SEQ ID NO: 10
- Backwards *primer:*

5'- GGAGTTCTCAAGCTCATCTCGAAGTGTTTTCTGTCGCTTATGAATACG
ACTCACTATAGGGTG -3'     APPENDED AS SEQ ID NO: 11

Underlined sequences complement *hph* in pAG32 (Goldstein and McCusker. 1999. Yeast 15:1541-1553) The rest are deleting sequences that complement regions flanking *PHO80* (SGD). The plasmid pAG32 (Goldstein and McCusker. 1999). containing the selectable marker hygromycin B phosphotransferase *(hph)* was used as template. FW primer complements to non-coding strain. Starting at ~ 483 bp upstream *hph* start (ATG) in pAG32 and 315 bp upstream *PHO80* start. BW primer complements to coding strand. Oligo, (63 bp.) starts 311 bp downstream *hph* end in pAG32. and 267 bp downstream *PHO80* end in S. cerevisiae.

The PCR mix consisted of Thermopol buffer, 0.1 µg/µl acetylated BSA (Bovine Serum Albumin), 0.2 mM dNTPs, 0.5 µM of each primer and 2 units of VENT polymerase. To each reaction approximately 0.7 µg/ml template DNA was added. Following the PCR reaction, products were separated by agarose get electrophoresis (0.7 % agarose in TBE buffer. 100V) and purified by QIAquick Gel Extraction Kit (QIAGEN).

Resulting PCR product used for deletion of *PHO80* by homologous recombination: 
CAGCGTATATTGGCTTTCCTTTAATCTAATGCCCCAAGCCCACATACGATTTA
GGTGACACTATAGAACGCGGCCGCCAGCTGAAGCTTCGTACGCTGCAGGTCG
ACGGATCCCCGGGTTAATTAAGGCGCGCCAGATCTGTTTAGCTTGCCTCGTCC
CCGCCGGGTCACCCGGCCAGCGACATGGAGGCCCAGAATACCCTCCTTGACA
GTCTTGACGTGCGCAGCTCAGGGGCATGATGTGACTGTCGCCCGTACATTTAG
CCCATACATCCCCATGTATAATCATTTGCATCCATACATTTTGATGGCCGCAC
GGCGCGAAGCAAAAATTACGGCTCCTCGCTGCAGACCTGCGAGCAGGGAAAC
GCTCCCCTCACAGACGCGTTGAATTGTCCCCACGCCGCGCCCCTGTAGAGAAA
TATAAAAGGTTAGGATTTGCCACTGAGGTTCTTCTTTCATATACTTCCTTTTAA
AATCTTGCTAGGATACAGTTCTCACATCACATCCGAACATAAACAACCATGGG
TAAAAAGCCTGAACTCACCGCGACGTCTGTCGAGAAGTTTCTGATCGAAA
AGTTCGACAGCGTCTCCGACCTGATGCAGCTCTCGGAGGGCGAAGAATC
TCGTGCTTTCAGCTTCGATGTAGGAGGGCGTGGATATGTCCTGCGGGTA
AATAGCTGCGCCGATGGTTTCTACAAAGATCGTTATGTTTATCGGCACTT
TGCATCGGCCGCGCTCCCGATTCCGGAAGTGCTTGACATTGGGGAATTC
AGCGAGAGCCTGACCTATTGCATCTCCCGCCGTGCACAGGGTGTCACGT
TGCAAGACCTGCCTGAAACCGAACTGCCCGCTGTTCTGCAGCCGGTCGC
GGAGGCCATGGATGCGATCGCTGCGGCCGATCTTAGCCAGACGAGCGGG
TTCGGCCCATTCGGACCGCAAGGAATCGGTCAATACACTACATGGCGTG
ATTTCATATGCGCGATTGCTGATCCCCATGTGTATCACTGGCAAACTGTG
ATGGACGACACCGTCAGTGCGTCCGTCGCGCAGGCTCTCGATGAGCTGA
TGCTTTGGGCCGAGGACTGCCCCGAAGTCCGGCACCTCGTGCACGCGGA
TTTCGGCTCCAACAATGTCCTGACGGACAATGGCCGCATAACAGCGGTC
ATTGACTGGAGCGAGGCGATGTTCGGGGATTCCCAATACGAGGTCGCCA
ACATCTTCTTCTGGAGGCCGTGGTTGGCTTGTATGGAGCAGCAGACGCG
CTACTTCGAGCGGAGGCATCCGGAGCTTGCAGGATCGCCGCGGCTCCGG
GCGTATATGCTCCGCATTGGTCTTGACCAACTCTATCAGAGCTTGGTTGA
CGGCAATTTCGATGATGCAGCTTGGGCGCAGGGTCGATGCGACGCAATC
GTCCGATCCGGAGCCGGGACTGTCGGGCGTACACAAATCGCCCGCAGAA
GCGCGGCCGTCTGGACCGATGGCTGTGTAGAAGTACTCGCCGATAGTGG
AAACCGACGCCCCAGCACTCGTCCGAGGGCAAAGGAATAATCAGTACTGA
CAATAAAAAGATTCTTGTTTTCAAGAACTTGTCATTTGTATAGTTTTTTTATAT
TGTAGTTGTTCTATTTTAATCAAATGTTAGCGTGATTTATATTTTTTTTCGCCTC
 
GACATCATCTGCCCAGATGCGAAGTTAAGTGCGCAGAAAGTAATATCATGCG
TCAATCGTATGTGAATGCTGGTCGCTATACTGCTGTCGATTCGATACTAACGC
CGCCATCCAGTGTCGAAAACGAGCTCGAATTCATCGATGATATCAGATCCACT
AGTGGCCTATGCGGCCGCGGATCTGCCGGTCTCCCTATAGTGAGTCGTATTCA
TAAGCGACAGAAAACACTTCGAGATGAGCTTGAGAACTCC. appended as SEQ
ID NO: 12
 Nucleotides in bold show the ORF of the *hgh* gene. Underlined sequences shows the PCR primers, partly complementary to the *PHO80* gene

The purified deletion fragment was transformed into YS18 by electroporation. The proteol for transformation was mainly based on a protocol at Gottschling Lab website (http://www.fhere.org/gottschling/yeast/ytrans.html). Transformants were incubated in 30°C in 1 ml 1M sorbitol + 1 ml 2x YPD for 2h to allow expression of the drug resistance marker, then spread onto selective growth medium (YPD. 0.9 mg/ml hygromycin B). Colonies appeared after a few days and putative transformants were tested for accuracy by PCR. The resulting strain was designated YD80 *(MAT*α: *his 3-11*, *3-15: leu2-3*, *2-112*; *ura3*Δ*5*: *canR: pho80*Δ::*hph*)

### 2.5. Construction of combined pho80Δ deletion and PHO4 and PHO5 overexpressing strains.

The plasmid pYX212 containing the insert *PHO5* (described above) was transformed into strain YD80 using a standard LiOAc *S*. *cerevisiae* transformation protocol. Furthermore. *PHO4* was PCR cloned as described above using the primers:

### Primers for overexpression of PHO4

- *Forward Primer* (*JP42*)
   5'-CGGAATTCATGGCCCGTACAACTTCTGAGG-3'. APPENDED AS SEQ ID NO: 7
- **Backwards primers (JT42)**
   5 -CGCTCGAGTCACGTGCTCACGTTCTGCAG-3'. APPENDED AS SEQ ID NO: 8

Underlined sequences complement EcoRI and XhoI restriction sites in pYX212 and the rest are sequences that complement start and end regions in *PHO4* (SGD).

Sequence of pYX212 containing the insert *PHO4:*

GAATTCATGGCCGTACAACTTCTGAGGGAATACACGGTTTTGTGGACG
ATCTAGAGCCCAAGAGCAGCATTCTTGATAAAGTCGGAGACTTTATCACC
GTAAACACGAAACGGCATGATGGGCGCGAGGACTTCAACGAGCAAAACG
ACGAGCTGAACAGTCAAGAGAACCACAACAGCAGTGAGAATGGGAACGA
GAATGAAAATGAACAAGACAGTCTCGCGTTGGACGACCTAGACCGCGCC
TTTGAGCTGGTGGAAGGTATGGATATGGACTGGATGATGCCCTCGCATG
CGCACCACTCCCCAGCTACAACTGCTACAATCAAGCCGCGGCTATTATAT
TCGCCGCTAATACACACGCAAAGTGCGGTTCCCGTAACCATTTCGCCGAA
CTTGGTCGCTACTGCTACTTCCACCACATCCGCTAACAAAGTCACTAAAA
ACAAGAGTAATAGTAGTCCGTATTTGAACAAGCGCAGAGGTAAACCCGG
GCCGGATTCGGCCACTTCGCTGTTCGAATTGCCCGACAGCGTTATCCCAA
CTCCGAAACCGAAACCGAAACCAAAGCAATATCCGAAAGTTATTCTGCCG
TCGAACAGCACAAGACGCGTATCACCGGTCACGGCCAAGACCAGCAGCA
GCGCAGAAGGCGTGGTCGTAGCAAGTGAGTCTCCTGTAATCGCGCCGCA
CGGATCGAGCCATTCGCGGTCGCTGAGTAAGCGACGGTCATCGGGCGCG
CTCGTGGACGATGACAAGCGCGAATCACACAAGCATGCAGAGCAAGCAC
GGCGTAATCGATTAGCGGTCGCGCTGCACGAACTGGCGTCTTTAATCCC
CGCGGAGTGGAAACAGCAAAATGTGTCGGCCGCGCCGTCCAAAGCGACC
ACCGTGGAGGCGGCCTGCCGGTACATCCGTCACCTACAGCAGAACGTGA
GCACGTGACTCGAGCTCAGCTAGCTAACTGAATAAGGAACAATGAACGTTTT
TCCTTTCTCTTGTTCCTAGTATTAATGACTGACCGATACATCCCTTTTTTTTTTT
GTCTTTGTCTAGCTCCAGCTTTTGTTCCCTTTAGTGAGGGTTAATTCAATTCAC
TGGCCGTCGTTTTACAACGTCGTGACTGGGAAAACCCTGGCGTTACCCAACTT
AATCGCCTTGCAGCACATCCCCCTTTCGCCAGCTGGCGTAATAGCGAAGAGGC
CCGCACCGATCGCCCTTCCCAACAGTTGCGCAGCCTGAATGGCGAATGGCGC
GACGCGCCCTGTAGCGGCGCATTAAGCGCGGCGGGTGTGGTGGTTACGCGCA
GCGTGACCGCTACACTTGCCAGCGCCCTAGCGCCCGCTCCTTTCGCTTTCTTCC
CTTCCTTTCTCGCCACGTTCGCCGGCTTTCCCCGTCAAGCTCTAAATCGGGGGC
TCCCTTTAGGGTTCCGATTTAGTGGTTTACGGCACCTCGACCCCAAAAAACTT
GATTAGGGTGATGGTTCACGTAGTGGGCCATCGCCCTGATAGACGGTTTTTCG
CCCTTTGACGTTGGAGTCCACGTTCTTTAATAGTGGACTCTTGTTCCAAACTGG
AACAACACTCAACCCTATCTCGGTCTATTCTTTTGATTTATAAGGGATTTTGCC
GATTTCGGCCTATTGGTTAAAAAATGAGCTGATTTAACAAAAATTTAACGCGA
ATTTTAACAAAATATTAACGTTTACAATTTCCTGATGCGGTATTTTCTCCTTAC
GCATCTGTGCGGTATTTCACACCGCATAGGGTAATAACTGATATAATTAAATT
GAAGCTCTAATTTGTGAGTTTAGTATACATGCATTTACTTATAATACAGTTTTT
TAGTTTTGCTGGCCGCATCTTCTCAAATATGCTTCCCAGCCTGCTTTTCTGTAA
CGTTCACCCTGTACCTTAGCATCCCTTCCCTTTGCAAATAGTCCTCTTCCAACA
ATAATAATGTCAGATCCTGTAGAGACCACATCATCCACGGTTCTATACTGTTG
ACCCAATGCGTCTCCCTTGTCATCTAAACCCACACCGGGTGTCATAATCAACC
AATCGTAACCTTCATCTCTTCCACCCATGTCTCTTTGAGCAATAAAGCCGATA
ACAAAATCTTTGTCGCTCTTCGCAATGTCAACAGTACCCTTAGTATATTCTCCA
GTAGATAGGGAGCCCTTGCATGACAATTCTGCTAACATCAAAAGGCCTCTAG

GTTCCTTTGTTACTTCTTCTGCCGCCTGCTTCAAACCGCTAACAATACCTGGCC
CCAGCACACCGTGTGCATTCGTAATGTCTGCCCATTCTGCTATTCTGTATACAC
CCGCAGAGTACTGCAATTTGACTGTATTACCAATGTCAGCAAATTTTCTGTCT
TCGAAGAGTAAAAAATTGTACTTGGCGGATAATGCCTTTAGCGGCTTAACTGT
GCCCTCCATCGAAAAATCAGTCAATATATCCACATGTGTTTTTAGTAAACAAA
TTTTGGGACCTAATGCTTCAACTAACTCCAGTAATTCCTTGGTGGTACGAACA
TCCAATGAAGCACACAAGTTTGTTTGCTTTTCGTGCATGATATTAAATAGCTT
GGCAGCAACAGGACTAGGATGAGTAGCAGCACGTTCCTTATATGTAGCTTTC
GACATGATTTATCTTCGTTTCCTGCAGGTTTTTGTTCTGTGCAGTTGGGTTAAG
AATACTGGGCAATTTCATGTTTCTTCAACACTACATATGCGTATATATACCAA
TCTAAGTCTGTGCTCCTTCCTTCGTTCTTCCTTCTGTTCGGAGATTACCGAATC
AAAAAAATTTCAAAGAAACCGAAATCAAAAAAAAGAATAAAAAAAAAAATGA
TGAATTGAATTGAAAAGCTGTGGTATGGTGCACTCTCAGTACAATCTGCTCTG
ATGCCGCATAGTTAAGCCAGCCCCGACACCCGCCAACACCCGCTGACGCGCC
CTGACGGGCTTGTCTGCTCCCGGCATCCGCTTACAGACAAGCTGTGACCGTCT
CCGGGAGCTGCATGTGTCAGAGGTTTTCACCGTCATCACCGAAACGCGCGAG
ACGAAAGGGCCTCGTGATACGCCTATTTTTATAGGTTAATGTCATGATAATAA
TGGTTTCTTAGACGTGCGGCCGCTCTAGAACTAGTGGATCAATTCCACGGACT
ATAGACTATACTAGTATACTCCGTCTACTGTACGATACACTTCCGCTCAGGTC
CTTGTCCTTTAACGAGGCCTTACCACTCTTTTGTTACTCTATTGATCCAGCTCA
GCAAAGGCAGTGTGATCTAAGATTCTATCTTCGCGATGTAGTAAAACTAGCTA
GACCGAGAAAGAGACTAGAAATGCAAAAGGCACTTCTACAATGGCTGCCATC
ATTATTATCCGATGTGACGCTGCAGCTTCTCAATGATATTCGAATACGCTTTG
AGGAGATACAGCCTAATATCCGACAAACTGTTTTACAGATTTACGATCGTACT
TGTTACCCATCATTGAATTTTGAACATCCGAACCTGGGAGTTTTCCCTGAAAC
AGATAGTATATTTGAACCTGTATAATAATATATAGTCTAGCGCTTTACGGAAG
ACAATGTATGTATTTCGGTTCCTGGAGAAACTATTGCATCTATTGCATAGGTA
ATCTTGCACGTCGCATCCCCGGTTCATTTTCTGCGTTTCCATCTTGCACTTCAA
TAGCATATCTTTGTTAACGAAGCATCTGTGCTTCATTTTGTAGAACAAAAATG
CAACGCGAGAGCGCTAATTTTTCAAACAAAGAATCTGAGCTGCATTTTTACAG
AACAGAAATGCAACGCGAAAGCGCTATTTTACCAACGAAGAATCTGTGCTTC
ATTTTTGTAAAACAAAAATGCAACGCGAGAGCGCTAATTTTTCAAACAAAGA
ATCTGAGCTGCATTTTTACAGAACAGAAATGCAACGCGAGAGCGCTATTTTAC
CAACAAAGAATCTATACTTCTTTTTTGTTCTACAAAAATGCATCCCGAGAGCG
CTATTTTTCTAACAAAGCATCTTAGATTACTTTTTTTCTCCTTTGTGCGCTCTAT
AATGCAGTCTCTTGATAACTTTTTGCACTGTAGGTCCGTTAAGGTTAGAAGAA
GGCTACTTTGGTGTCTATTTTCTCTTCCATAAAAAAAGCCTGACTCCACTTCCC
GCGTTTACTGATTACTAGCGAAGCTGCGGGTGCATTTTTTCAAGATAAAGGCA
TCCCCGATTATATTCTATACCGATGTGGATTGCGCATACTTTGTGAACAGAAA
GTGATAGCGTTGATGATTCTTCATTGGTCAGAAAATTATGAACGGTTTCTTCT
ATTTTGTCTCTATATACTACGTATAGGAAATGTTTACATTTTCGTATTGTTTTC
GATTCACTCTATGAATAGTTCTTACTACAATTTTTTTGTCTAAAGAGTAATACT
AGAGATAAACATAAAAAATGTAGAGGTCGAGTTTAGATGCAAGTTCAAGGAG
CGAAAGGTGGATGGGTAGGTTATATAGGGATATAGCACAGAGATATATAGCA
AAGAGATACTTTTGAGCAATGTTTGTGGAAGCGGTATTCGCAATATTTTAGTA
GCTCGTTACAGTCCGGTGCGTTTTTGGTTTTTTGAAAGTGCGTCTTCAGAGCGC
TTTTGGTTTTCAAAAGCGCTCTGAAGTTCCTATACTTTCTAGAGAATAGGAAC
TTCGGAATAGGAACTTCAAAGCGTTTCCGAAAACGAGCGCTTCCGAAAATGC
AACGCGAGCTGCGCACATACAGCTCACTGTTCACGTCGCACCTATATCTGCGT
GTTGCCTGTATATATATATACATGAGAAGAACGGCATAGTGCGTGTTTATGCT

TAAATGCGTACTTATATGCGTCTATTTATGTAGGATGAAAGGTAGTCTAGTAC
CTCCTGTGATATTATCCCATTCCATGCGGGGTATCGTATGCTTCCTTCAGCACT
ACCCTTTAGCTGTTCTATATGCTGCCACTCCTCAATTGGATTAGTCTCATCCTT
CAATGCTATCATTTCCTTTGATATTGGATCATATGCATAGTACCGAGAAACTA
GTGCGAAGTAGTGATCAGGTATTGCTGTTATCTGATGAGTATACGTTGTCCTG
GCCACGGCAGAAGCACGCTTATCGCTCCAATTTCCCACAACATTAGTCAACTC
CGTTAGGCCCTTCATTGAAAGAAATGAGGTCATCAAATGTCTTCCAATGTGAG
ATTTTGGGCCATTTTTTATAGCAAAGATTGAATAAGGCGCATTTTTCTTCAAA
GCTGCGGCCGCACTCTCACTAGTACGTCAGGTGGCACTTTTCGGGGAAATGTG
CGCGGAACCCCTATTTGTTTATTTTTCTAAATACATTCAAATATGTATCCGCTC
ATGAGACAATAACCGTGATAAATGCTTCAATAATATTGAAAAAGGAAGAGTA
TGAGTATTCAACATTTCCGTGTCGCCCTTATTCCCTTTTTTGCGGCATTTTGCC
TTCCTGTTTTTGCTCACCCAGAAACGCTGGTGAAAGTAAAAGATGCTGAAGAT
CAGTTGGGTGCACGAGTGGGTTACATCGAACTGGATCTCAACAGCGGTAAGA
TCCTTGAGAGTTTTCGCCCCGAAGAACGTTTTCCAATGATGAGCACTTTTAAA
GTTCTGCTATGTGGCGCGGTATTATCCCGTATTGACGCCGGGCAAGAGCAACT
CGCTCGCCGCATACACTATTCTCAGAATGACTTGGTTGAGTACTCACCAGTCA
CAGAAAAGCATCTTACGGATGGCATGACAGTAAGAGAATTATGCAGTGCTGC
CATAACCATGAGTGATAACACTGCGGCCAACTTACTTCTGACAACGATCGGA
GGACCGAAGGAGCTAACCGCTTTTTTGGACAACATGGGGGATCATGTAACTC
GCCTTGATCGTTGGGAACCGGAGCTGAATGAAGCCATACCAAACGACGAGCG
TGACACCACGATGCCTGTAGCAATGGCAACAACGTTGCGCAAACTATTAACT
GGCGAACTACTTACTCTAGCTTCCCGGCAACAATTAATAGACTGGATGGAGG
CGGATAAAGTTGCAGGACCACTTCTGCGCTCGGCCCTTCCGGCTGGCTGGTTT
ATTGCTGATAAATCTGGAGCCGGTGAGCGTGGGTCTCGCGGTATCATTGCAGC
ACTGGGGCCAGATGGTAAGCCCTCCCGTATCGTAGTTATCTACACGACGGGG
AGTCAGGCAACTATGGATGAACGAAATAGACAGATCGCTGAGATAGGTGCCT
CACTGATTAAGCATTGGTAACTGTCAGACCAAGTTTACTCATATATACTTTAG
ATTGATTTAAAACTTCATTTTTAATTTAAAAGGATCTAGGTGAAGATCCTTTTT
GATAATCTCATGACCAAAATCCCTTAACGTGAGTTTTCGTTCCACTGAGCGTC
AGACCCCGTAGAAAAGATCAAAGGATCTTCTTGAGATCCTTTTTTTCTGCGCG
TAATCTGCTGCTTGCAAACAAAAAAACCACCGCTACCAGCGGTGGTTTGTTTG
CCGGATCAAGAGCTACCAACTCTTTTTCCGAAGGTAACTGGCTTCAGCAGAGC
GCAGATACCAAATACTGTCCTTCTAGTGTAGCCGTAGTTAGGCCACCACTTCA
AGAACTCTGTAGCACCGCCTACATACCTCGCTCTGCTAATCCTGTTACCAGTG
GCTGCTGCCAGTGGCGATAAGTCGTGTCTTACCGGGTTGGACTCAAGACGATA
GTTACCGGATAAGGCGCAGCGGTCGGGCTGAACGGGGGGTTCGTGCACACAG
CCCAGCTTGGAGCGAACGACCTACACCGAACTGAGATACCTACAGCGTGAGC
TATGAGAAAGCGCCACGCTTCCCGAAGGGAGAAAGGCGGACAGGTATCCGGT
AAGCGGCAGGGTCGGAACAGGAGAGCGCACGAGGGAGCTTCCAGGGGGAAA
CGCCTGGTATCTTTATAGTCCTGTCGGGTTTCGCCACCTCTGACTTGAGCGTCG
ATTTTTGTGATGCTCGTCAGGGGGGCGGAGCCTATGGAAAAACGCCAGCAAC
GCGGCCTTTTTACGGTTCCTGGCCTTTTGCTGGCCTTTTGCTCACATGTTCTTTC
CTGCGTTATCCCCTGATTCTGTGGATAACCGTATTACCGCCTTTGAGTGAGCT
GATACCGCTCGCCGCAGCCGAACGACCGAGCGCAGCGAGTCAGTGAGCGAG
GAAGCGGAAGAGCGCCCAATACGCAAACCGCCTCTCCCCGCGCGTTGGCCGA
TTCATTAATGCAGCTGGCACGACAGGTTTCCCGACTGGAAAGCGGGCAGTGA
GCGCAACGCAATTAATGTGAGTTACCTCACTCATTAGGCACCCCAGGCTTTAC
ACTTTATGCTTCCGGCTCCTATGTTGTGTGGAATTGTGAGCGGATAACAATTT
CACACAGGAAACAGCTATGACCATGATTACGCCAAGCTCGAAATACGACTCA

Bolded sequence shows *PHO4*
Underlined sequences represent sites for primers.

Also pYX212 with *PHO4* was transformed into YD80 using the methods already described.

### Sample preparation, inositol phosphates and pNPP

1.5 ml of cell suspension was withdrawn at intervals during the growth and centrifuged for 3 minutes at 13000 rpm to pellet the cells. One ml of the supernatant was transferred to a new micro-tube and acidified by adding 150 µl of 4 M HCl. This yields approximately 0.5 M HCl, which is enough to inactivate all enzymatic activity potentially left in the supernatant. Chemical acid hydrolysis of inositol phosphates does not take place in such solutions without strong heating. These samples were stored in freezer until use for analysis of IP:s and pNPP. A reference sample for identification of peaks was prepared by chemically hydrolyzing 1 mM sodium phytate (Na-IP6) in 2 ml of 0.5 M HCl. The solution was heated to 110°C for 15 h yielding a mixture of isomers of IP:s that was stored in freezer until used as reference sample.

### Bread dough making, phosphate extraction and analysis

To analyze the Pᵢ and IP6 content in a typical environment, baker's yeast are exposed to during bread leavening two simple doughs were mixed, in which two types of flour were used: whole meal wheat flour (100%) extraction rate: i.e. the proportion of the whole wheat grain obtained as finished flour), and normal wheat flour (extraction rate 60%). The dough mixture contained: 300g flour, 10g sucrose, 3g NaCl, 20g commercial baker's yeast (Swedish Yeast Company) and 200g tap-water. At time zero and thereafter every 15 min, 20 g of dough was withdrawn and mixed with 40 ml 0.5 M HCl (at room temperature this HCl concentration will not hydrolyze IP6, but is sufficient to inactivate enzymatic activity). Inorganic phosphate (Pᵢ) and IP6 were extracted by magnetically stirring the mixture for three hours at room temperature. The extracts were centrifuged the supernatant collected and frozen until use. For analysis, the samples were thawed and 1 ml was centrifuged in a micro-tube for 5 min at 13000 x g. The resulting supernatants were appropriately diluted with mQ-water and inositol phosphates were analyzed by HPIC chromatography as described in previous paragraph, the ion chromatographic analysis of Pᵢ were performed using a Dionex (Sunnyvate. CA. USA) model 4500i equipped with a 50 µl loop injector, PAX-100 guard and analytical column, Anion Micro Membrane Supressor and a conductivity detector. The samples were eluted with a linear gradient of water and increasing portion of 200 mM NaOH, starting with 6 % NaOH and ending after 35 min at 50 % NaOH.

### Results

### Expression and repression of phytase activity in wild-type yeasts

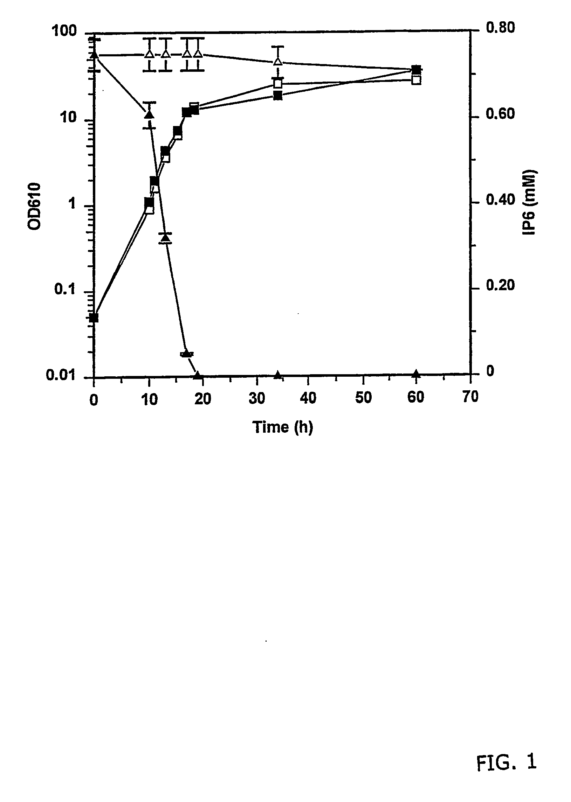

IP6 is efficiently degraded by yeast in a synthetic medium with IP6 as the sole phosphorous source. Türk et al. (2000) Inositol hexaphosphate hydrolysis by baker's yeast. Capacity, kinetics, and degradation products. J. Agric. Food Chem. 48. 100-104 In order to test whether extracellular IP6 hydrolysis by *S. cerevisiae* was repressed by inorganic phosphate (Pᵢ), synthetic yeast minimal medium containing Pᵢ (26 mM) was supplemented with IP6 (CBSIP6+Pᵢ). For all *S. cerevisiae* tested, that is SKQ2n. CBS 7764. CBS 7765, YS18, BY4741 and YMR4 virtually no breakdown of IP6 was detected in the IP6+Pᵢ cultures, showing an efficient repression of phytase activity by high levels of extracellular Pᵢ. Referring to FIG. 1. growth of *S. cerevisiae* SKQ2n (squares) and extracellular concentration of inositol hexaphosphate (IP6: triangles), measured by HPIC, as a function of time is shown. Growth was performed in synthetic CBS medium with 2% (wt/vol) glucose as carbon and energy source. Filled symbols: Inositol hexaphosphate (IP6) as the sole phosphorous source: open symbols: inositol hexaphosphate plus inorganic phosphate (Pᵢ) as combined phosphorous sources. Data are from a representative experiment performed many times. Error bars on IP6 data show maximum variation between repealed HPIC analysis. The typical pattern for the IP6 concentration in the medium during growth in the absence and presence of Pᵢ is depicted for strain SKQ2n in FIG.1.

In this experiment, all the IP6 was depleted before 20h of growth in the CBSIP6 culture, white in the CBSIP6+Pᵢ culture no IP6 was degraded at that time. For all strains, the growth rates, as calculated by regression in the exponential respiro-fermentative phase (glucose growth), were as rapid in CBSIP6 medium as compared with CBSIP6+Pᵢ or CBSPᵢ media, showing efficient utilization of IP6 as P-source for growth by *S. cerevisiae*.

The same type of experiments were performed also in complex and rich YPD medium, either with the addition of only IP6 (YPDIP6) or with the addition of IP6 and Pᵢ (YPDIP6+Pᵢ). The YPD experiments yielded data consistent with the CBS cultures that is IP6 degradation without loss in growth rate in YPDIP6 medium and repression of phytase activity in YPDIP6+Pᵢ medium (data not shown). However, the Pᵢ induced repression was for some strains (e.g. YS18) less pronounced in YPD as compared with CBS medium, that is a certain constitutive, not negligible, phytase activity was detected.

### Inositol phosphates formed during IP6 degradation

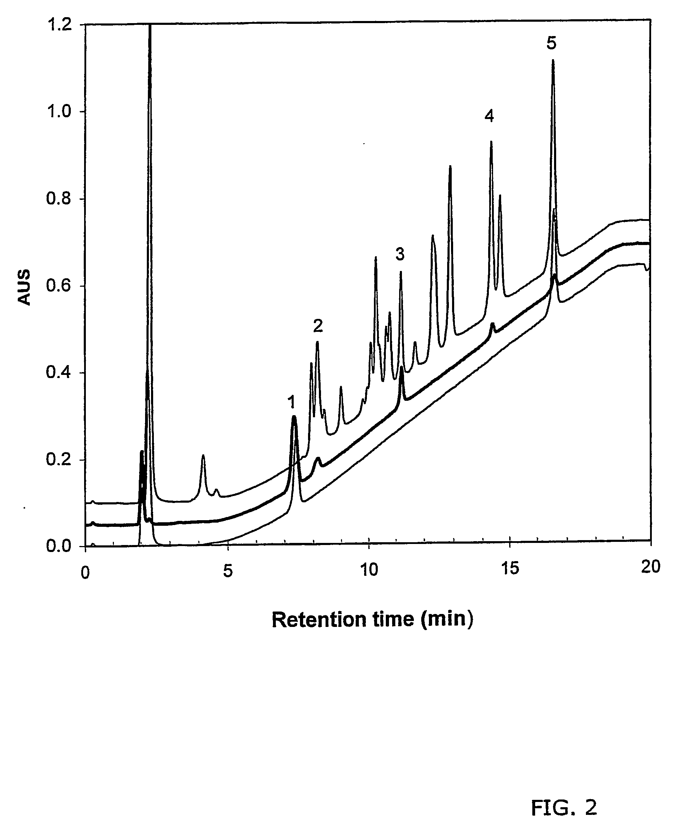

IP6 and the breakdown products formed as a result of IP6 hydrolysis by the yeast were identified and quantified by HPIC. Referring to FIG. 2. chromatographic profiles (HPIC) of chemical acid hydrolysate of sodium phytate (first line), and supernatant from *S. cerevisiae* cultures (second and third lines) after 15 h of growth is shown. *S. cerevisiae* was cultured in CBS medium containing either IP6 as the sole P-source (second line, thick) demonstrating yeast phytase activity, or in CBS medium with IP6 and Pᵢ as combined P-sources (third line) showing Pᵢ-induced repression of phytase activity. Numbered peaks: 1: SO₄⁻²: 2: Ins(1.2.6)P₃: 3: Ins(1.2.5.6)P₄: 4: Ins(1.2.4.5.6)P₅: IP6.

The chromatographic profile showed that the enzymes exerting the yeast phytase activity are very specific with respect to position on the inositol ring. Only one isomer of each IP₅. IP₄ and IP₃ was detected for samples withdrawn during growth of *S*. *cerevisiae* in CBSIP6 medium (FIG. 2, second line). These isomers maligned with an acid chemical hydrolysate (FIG.2, first line, in which all peaks previously have been identified) proved to be I(1,2,4,6)P₅, I(1,2,5,6)P₄, I(1,2,6)P₃, respectively. It is known that peak 2 in the hydrolysate may contain a mixture of I(1,2,6)P₃ and I(1,2,3)P₃. However, the I(1,2,3)P₃ isomer can be excluded in the yeast samples since position 3 was already missing. The same pattern of IP isomers was obtained for all strains tested in our collection. including laboratory strains and several wild-types. The lower. third line in FIG.2 shows the extracellular composition of IP:s from a *S*. *cerevisiae* CBSIP6+Pi culture at 15 hrs of growth. In such cultures no lower inositol phosphates were detectable and the concentration of IP6 (peak 5) remained high through out the experiment proving the Pᵢ induced repression of extracellular phytase activity.

### PHO deletion mutants

The major secretory acid phosphatase. encoded by *PHO5*. may be involved in phytate hydrolysis. A *PHO5* deletion mutant may be less efficient in degrading extracellular IP6 as compared with the corresponding parent strain. For this purpose the *pho3*Δ*pho5*Δ double mutant YMR4 was used. By using this mutant the effect of missing *PHO3.* in addition to *PHO5*. was assessed. However, this gene may be less important since it has been shown to be repressed by thiamine. (which is present in the CBS medium) and its product is mainly a thiamine phosphatase. Praekelt et al. (1994) Regulation of THI4 (MOL1). a thiamine-biosynthetic gene of Saccharomyces cerevisiae. Yeast. 10.481-490. Contrary to expectation, the mutant was not affected.

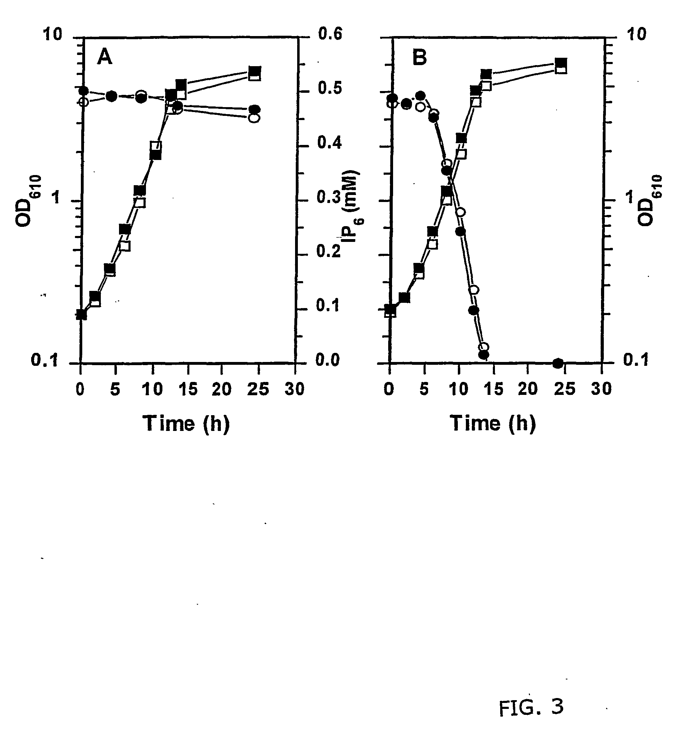

Referring to FIGs. 3A and 3B. growth (squares) of *S*. *cerecisiae* parent strain YS18 (open symbols) and *S*. *cerevisiae* YMR4 *pho5Δpho3Δ* double deletion mutant (filled symbols) and extracellular concentration of IP6 (circles) as determined by HPIC. FIG. 3A shows synthetic CBS medium with glucose as carbon and energy source and IP6 plus Pᵢ as phosphorous sources (repressing conditions). FIG. B shows synthetic CBS medium with glucose as carbon and energy source and IP6 as the sole phosphorous source (de-repressing conditions). Represented data are means of double samples from a representative experiment run twice.

In repeated experiments strain YMR4 hydrolyzed extracellular IP6 at a rate equal to its parent strain YS18 (FIG. 3B). In addition. Figure 3A shows that in CBSIP6 + Pᵢ medium (repressing conditions), the pattern of growth and the Pᵢ induced repression was unaffected in the mutant strain YMR4, which closely follows the pattern for YS18. For both strains at time 24 h after inoculation. 94% of the initial amount of IP6 was still present in the CBSIP6+P_{¡} cultures (FIG. 3A). At this time. neither IP6 nor any other lower IP:s were detectable in the medium of the parallel CBSIP6 culture (FIG. 3B). Additional evidence for independence of *PHO5* in this context was provided by the rate of growth for the mutant in CBSIP6 medium. The calculated growth rate in the exponential respiro-fermentative phase (growth on glucose. CBSIP6 medium) was 0.33h⁻¹ (generation time of 2.1 h) for both YMR4 and its parent strain YS18. This is not evidence that Pho5p and Pho3p are unable to hydrolyze IP6. it is only evidence that shows that they are not needed by the yeast for intact phytase activity.

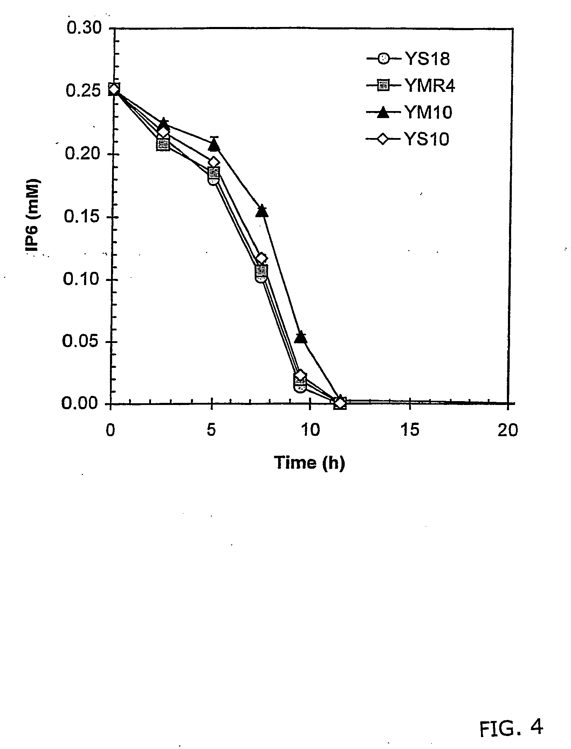

By PCR and homologous recombination *PHO10* was deleted in YMR4 and YS18 thereby obtaining a *pho5* Δ*pho3* Δ*pho10*Δ triple mutant and pho10Δ single mutant labeled YM10 and YS10. respectively. Referring to FIG. 4. concentration of inositol hexaphosphate (IP6) in yeast culture supernatant as a function of growth in CBS medium with IP6 as the sole phosphorous source is shown. Grey circles: YS18 parent strain with all *PHO* genes intact: filled squares: YMR4 *pho3* Δ*pho5* Δ double mutant: filled triangles: YM10 *pho3* Δ*pho5* Δ*pho10*Δ triple mutant: and open diamonds: YS10 *pho10*Δ single deletion mutant. Data are means of double samples from separate cultures and error bars show the variation between those. These strains were cultured in CBSIP6 and YPDIP6 medium, and the medium concentration of IP6 was monitored by HPIC during growth. In CBS. the rate of IP6 degradation was unaffected in YS10 showing that also *PHO10* was superfluous (FIG. 4) for intact phytase activity.

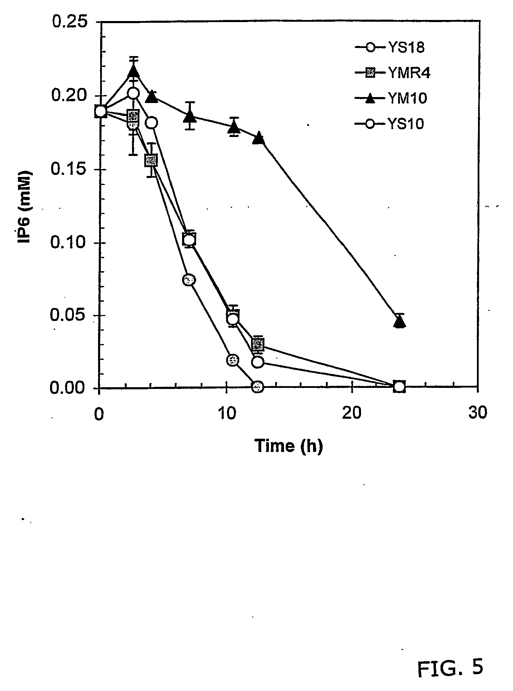

Even in YM10. in which the only remaining secretory phosphatase is Pho11p. the net extracellular phytase activity was unaffected as compared with YS18 and YMR4. Only a slight. but significant, decrease in the rate of IP6 degradation was in CBS detected in this mutant (FIG. 4). However, in the complex medium YPD. the triple mutant showed a strong reduction in rate ot extracellular IP6 (FIG. 5) with maintained growth rate. suggesting phytase activity for Pho10p and at least one more enzyme.

Referring to FIG. 5. concentration of inositol hexaphosphate (IP6) in yeast culture supernatant as a function of growth in complex YPD medium with IP6 as the added phosphorous source is shown. Grey circles: YS18 parent strain with all *PHO* genes intact: filled squares: YMR4 *pho3Δpho5*Δ double mutant: filled triangles: YM10 *pho3* Δ*pho5* Δ*pho10*Δ triple mutant: and open diamonds: YS10 *pho10*Δ single deletion mutant. Data are means of double samples from separate cultures and error bars show the variation between those.

### Overexpression of PHO5

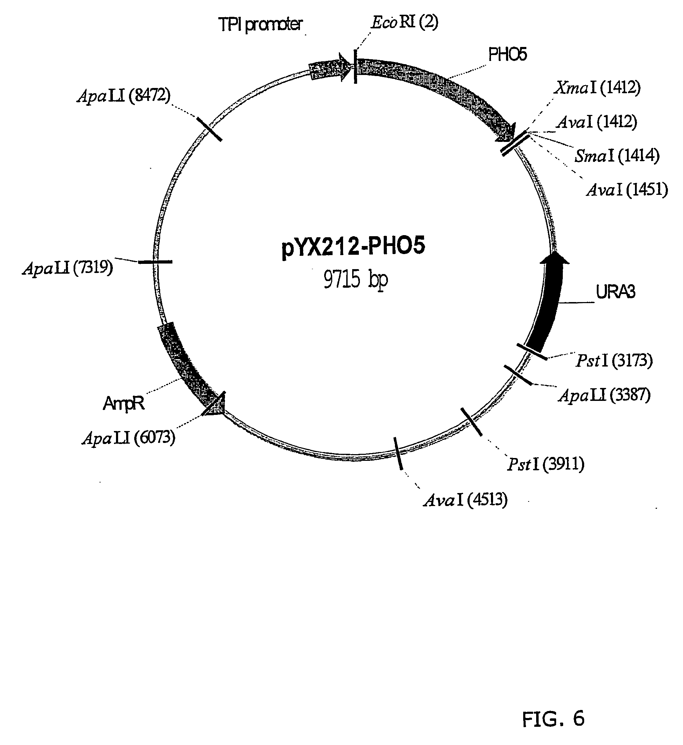

Referring to FIG. 6. a vector construct consisting of plasmid pYX212 containing the insert *PHO5* used for transformation o YS18 and YMR4. The glycolytic promoter triose phosphate isomerase (*TP11*) controlling expression of *PHO5*. the yeast selection marker *URA3* and the Amp^{R} gene are depicted.
In order to assess the impact of exclusively *PHO5* expression on the extracellular IP6 degradation YMR4 and YS18 were both transformed with pYX212 containing the insert *PHO5* controlled by the constitutive glycolytic promoter TP11 (FIG. 6).

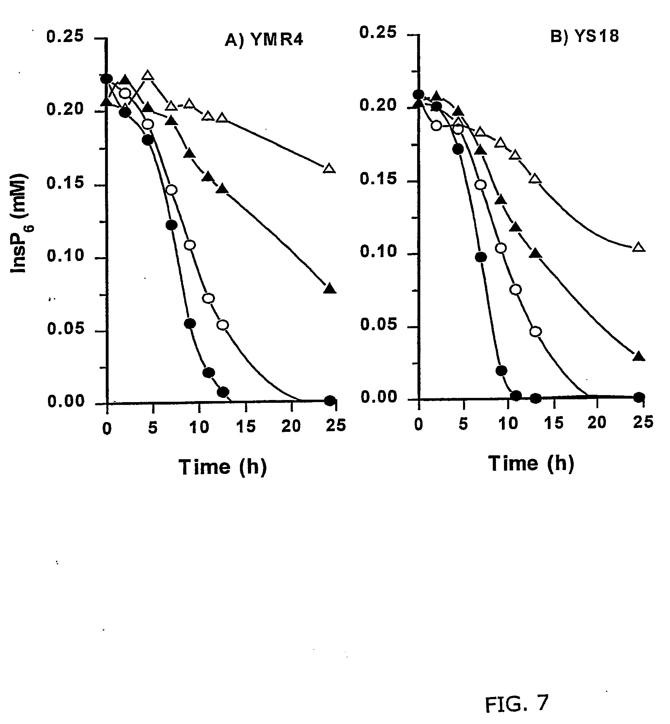

The resulting, strains were designated YM-p5 and YS-p5. respectively (Table 1). The experiments with these mutants were performed in YPD medium with the addition of the appropriate phosphorous source. Growth and extracellular IP6 concentration were assessed as a function of time. Referring to FIGs. 7A and 7B. extracellular concentration of IP6 as a function of growth time of *S*. *cerevisiae* YMR4 (A) and YS18 (B). with and without the *PHO5* containing plasmid pYX212. Open symbols: strains without plasmid; solid symbols: strains containing the plasmid pYX212-*PHO5*. The experiment was performed in YPD medium with 2% glucose supplemented with either only IP6 (circles) or with both IP6 and Pᵢ (triangles). Represented data are means from two to three independent experiments with a standard deviation never exceeding 5%. From both YM-p5 and YS-p5 data it is evident that *PHO5* encodes an enzyme with phytase activity ( FIGs. 7A and B).

Under normally repressing conditions (YPDIP6+Pi) IP6 was by YM-p5 and YS-p5 degraded at a fairly high rate (filled triangles in FIGs. 7A and 7B). demonstrating that phytase activity was in these mutants constitutively expressed. The corresponding parent strains lacking the plasmid are shown as controls (open triangles). These controls were in YPD somewhat lesser repressed as compared with CBS medium, however, much more repressed than the cells with plasmid. After 24 h of growth strain YM-p5 had degraded 63 % of the initial IP6 whereas in the YMR4 control strain the corresponding value at 24 h was only 22.5 % (Fig. 6). The rate of IP6 degradation in YPDIP6 non-repressing medium was also analyzed. During these conditions *PHO10* and *PHO11* plus the introduced *PHO5* would be expressed in YM-p5. and *PHO5*. *PHO10* and *PHO11* plus the introduced *PHO5* in the YS-p5 strain. The data show that the plasmid located *PHO5* indeed increased the net rate of IP6 degradation (FIGs. 7A and 7β) in both strains. The effect was more pronounced in YS-p5. in which the *PHO5* gene is present both in the genome and on the plasmid. The combined data from the *PHO5* overexpression experiments and the experiments with deletion strains compared with their parent strains (FIGs. 3 and 4) shows that Pho5p. Pho10p and at least one more enzyme, believed to be Pho11p. are by definition all phytases.

### Deletion of the regulatory genes PHO80. PHO85 and PHO2

The *PHO* system is involved in the yeast phytase activity. Accordingly. deletions in the regulatory genes *PHO80* and *PHO85* yield constitutive phytase activity.

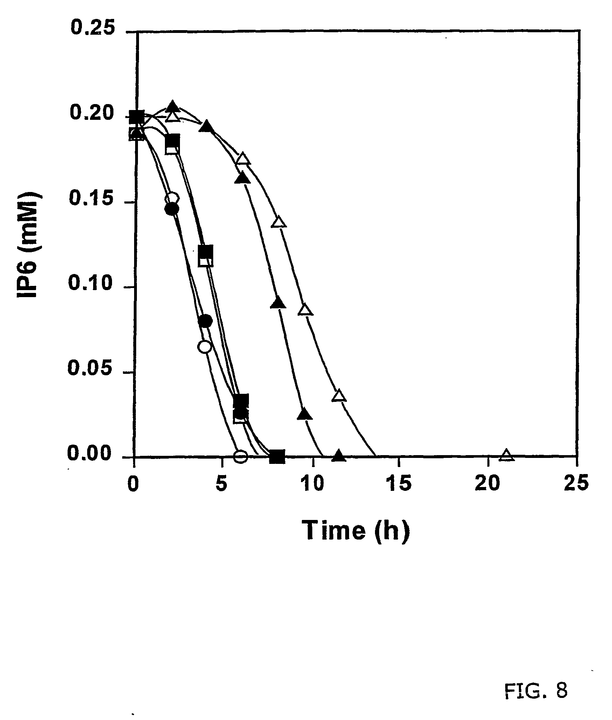

Referring to FIG. 8. a direct comparison of phytate degrading capacity between *PHO5* overexpressing strains, and *pho80*Δ and *pho85*Δ deletions mutants. Extracellular concentration of IP6 is plotted as a function of growth time of *S*. *cerevisiae* YS18-p5 (triangles) with the *PHO5* containing plasmid pYX212. *S*. *cerevisiae pho80Δ deletion* mutant (squares) and *pho85*Δ deletion mutant (circles) is shown. The experiment was performed in YPD medium with 2% glucose supplemented with either only IP6 (solid symbols) or with both IP6 and Pᵢ (open symbols). Data are means from two independent experiments with a standard deviation never exceeding 5%.

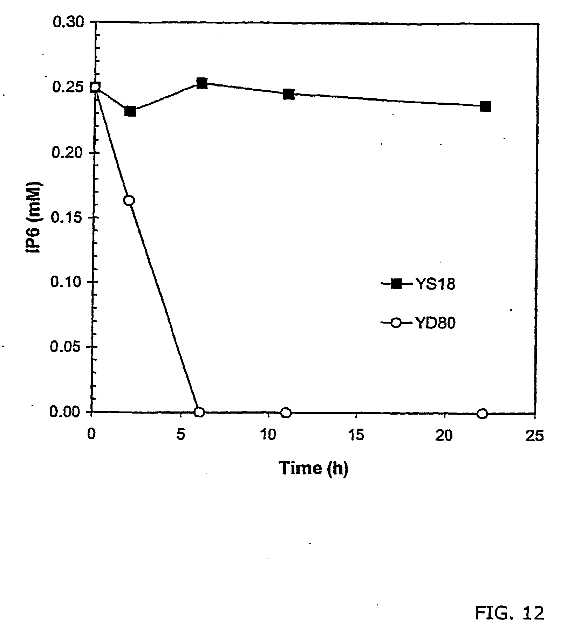

As shown in FIG. 8 these mutants were highly effective in degrading IP6. and completely constitutive demonstrating that the *PHO* system indeed is involved in *S*. *cerevisiae* phytase activity. Since the net rate of IP6 degradation was very rapid, these strains were compared with the YS18-p5 (*PHO5* overexpressing) strain. Both strain *pho80Δ* and *pho85Δ* showed a higher net rate in IP6 degradation as compared with the YS18-p5 (FIG. 8). The growth rate was unchanged in strain *pho80*Δ. however significantly reduced in strain *pho85Δ.* In addition to the *pho80*Δ and *pho85*Δ strains retrieved from BY4741 (EUROSCARF collection). another *pho80*Δ, *referring ro* *Fig. 12**.* mutant was constructed by deletion in YS 18. yielding strain YD80. The highly efficient extracellular phytase activity was reconfirmed in this strain.

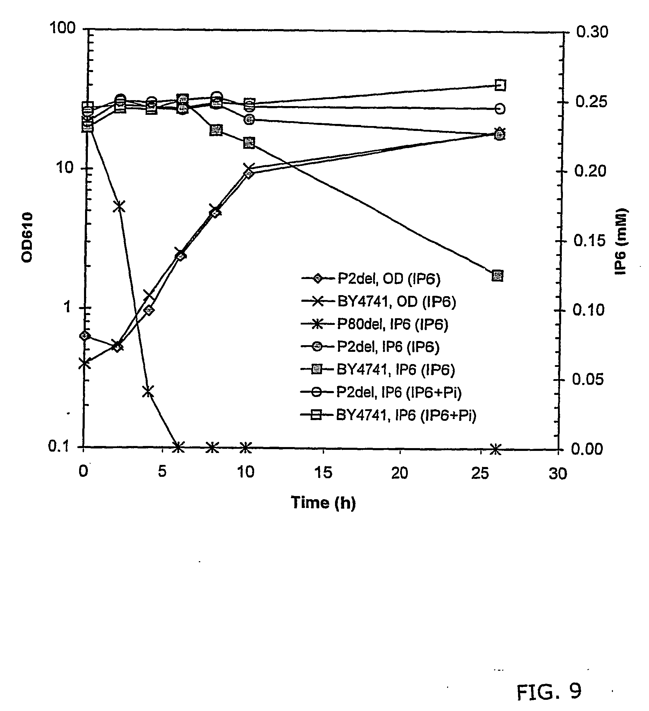

Referring to FIG. 9. phytate degrading capacity of *pho2*Δ deletion mutant is shown. For comparison. *pho80*Δ deletion strain and BY4741 with all PHO genes intact are also shown. Extracellular concentration of IP6 is plotted as a function of growth time in YPD. For explanation of symbols see Figure. The P-source used is shown within parenthesis. In addition to IP6 data. growth data as OD610. are shown for the *pho2*Δ deletion mutant and for its parent strain BY4741. Data are means from two independent experiments with a standard deviation never exceeding 5%.

*PHO2* is together with *PHO4* known to positively regulate the expression of the secretory phosphatases PHO5. PHO10 and PHO11. In a direct comparison the *pho2* deletion mutant was demonstrated to lack virtually all phytase activity. demonstrating that yeast phytase activity is largely a matter of its *PHO* system. The phytase activity may therefore be further increased by overexpressing *PHO2* and/or *PHO4.*

### Expression of PHO3

The expression of *PHO3.* in addition to expression of *PHO5. PHO10* and *PHO11* may increase the rate of extracellular IP6 degradation. For this purpose the parent strain YS18 was used in a CBS medium excluding thiamine. which is known to repress *PHO3* expression. Nosaka et al. (1989) A possible role for acid phosphatase wich thiamin-binding activity encoded by PHO3 in yeast. FEMS Microbiol. Lett. 51. 55-59: Nosaka (1990) High affinity of acid phosphatase encoded by PHO3 gene in Saccharomyces cerevisiae for thiamin phosphates. Biochim. Biophys. Acta. 1037. 147-154. As controls normal CBSIP6 medium (with thiamine) was used. with and without thiamine phosphate to ensure that the yeast was not negatively affected by the missing thiamine. Growth rates and IP6 degradation were by YS18 not different between the three media used, indicating that *PHO3* is of no or minor significance for yeast phytase activity (data not shown).

### Competitive degradation of IP6 and pNPP

The PHO system in *S*. *cerevisiae* has frequently been studied using phenyl phosphate and para-nitrophenyl phosphate (pNPP) as substrates. Monod et al. (1989) Functional analysis of the signal-sequence processing site of yeast acid phosphatase. Eur. J. Biochem. 182. 213-221; Shnyreva et al. (1996) Biochemical properties and excretion behavior of repressible acid phosphatases with altered subunit composition. Microbiol. Res. 151.291-300: Martinez et al. (1998) Identification, cloning and characterization of a derepressible Na -coupled phosphate transporter in Sacccharomyces cerevisiae. Mol. Gen. Genet. 258. 628-638. These compounds are used as model organic phosphorous source. Yeasts are often exposed to several organic phosphorous sources.

Accordingly. the impact of adding a second organic phosphorous compound (pNPP) on the rate of IP6 degradation was studied. Both compounds are readily degraded by *S*. *cerevisiae* and since the PHO system is involved in both cases a competitive situation arose.

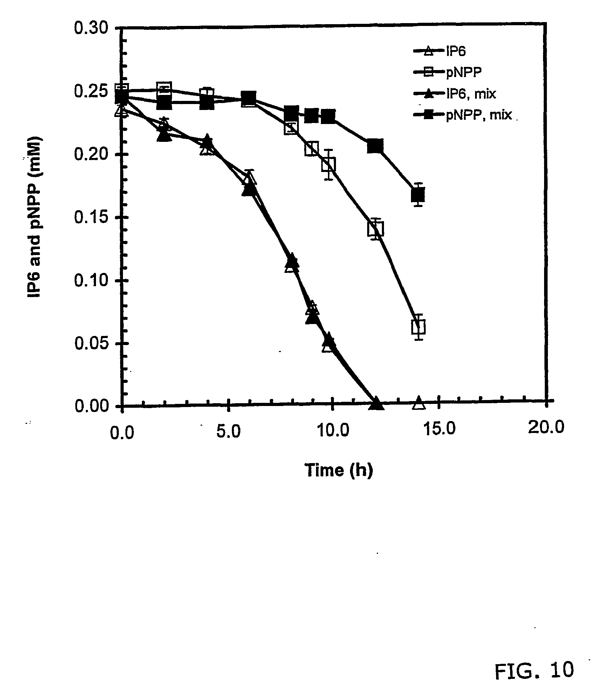

Referring to FIG. 10. extracellular concentration of para-nitrophenyl phosphate (pNPP. squares) and IP6 (triangles) as a function of growth of *S*. *cerevisiae* SKQ2n in CBS medium with 2% glucose as carbon and energy source is shown. The CBS medium was supplemented either with IP6 as the sole P-source (open triangles). with pNPP as the sole P-source (open squares) or with both IP6 and pNPP as combined P-sources (Solid symbols).

In the experiment. with both IP6 and pNPP present and Pᵢ absent. the rate of IP6 degradation was completely unchanged as compared with IP6 alone (FIG. 10). However. the rate of pNPP was delayed by presence of IP6 compared to pNPP alone. Most of the degradation of pNPP occurred after IP6 was depleted from the medium. At 12 h of growth. IP6 was depleted in both cultures, whereas only 17 % of the initial pNPP was used in the mixed culture, and 49% in the sole pNPP culture. The experiment was also performed with the pNPP concentration set to 6 times the concentration of IP6 (2.25 mM) to obtain equal stoichiometry in number of available phosphate groups. Six times more frequently pNPP randomly met the enzymes. The experiment yielded similar data. that is no change in rate of IP6 hydrolysis in the presence of pNPP and delayed pNPP degradation as compared with pNPP alone was observed (data not shown).

### Content of inorganic phosphate and IP6 in bread dough

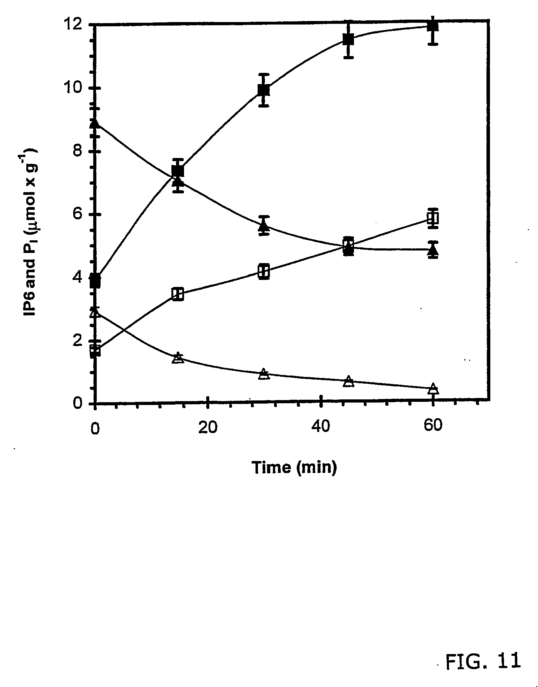

To understand the yeast environment during bread making. relative to the PHO system. the Pᵢ and IP6 content were analyzed in two types of wheat based bread doughs. In both doughs the level of Pᵢ increased and the level of IP6 decreased as a function of leavening time (FIG.11).

Referring to FIG. 11. the content of inositol hexaphosphate (IP6. triangles) and inorganic phosphate (Pᵢ: squares), expressed as µmol per gram wet dough. as a function of leavening time in two types of wheat based doughs is shown. Solid symbols: whole meal wheat flour (100% extraction rate: i.e. the proportion of the whole wheat grain obtained as finished flour). and open symbols: white wheat flour (extraction rate 60%). Error bars indicate the maximum variation between double samples.

The concentration of Pᵢ was higher in the whole wheat flour dough starting at 4 µmol/g wet dough (approximately equal to mM). and ending after 60 min of leavening at 11.8 µmol/g dough. white in the white wheat flour the start and end concentrations were 1.7 µmol/g and 5.8 µmol/g. respectively. The initial concentrations of IP6 were 9 µmol/g and 3 µmol/g for whole-wheat flour and white wheat flour. respectively. decreasing to 4.8 and 0.4 µmol/g after 60 minutes of leavening. This demonstrates that phytases are active in the dough.

Most previous investigations in the context of yeast degrading IP6 refer to a "yeast phytase". frequently distinguishing it from phosphatases. without identifying genes and enzymes. Nayini et al. (1984) The phytase of yeast. Lebensm. Wiss. u-Technol. 17. 24-26; Harland et al. (1989) Effects of phytase from three yeasts on phytate reduction in Norwegian whole wheat flour. Cereal Chem. 4. 357-358: Nair et al. (1991) Phytic acid content reduction in canola meal by various microorganisms in a solid state fermentation process. Acta Biotechnologica. 11. 211-218. Lambrechts et al. (1992) Utilization of phytate by some yeasts. Biotechnol. Lett. 14. 61-66 Attempts to assess the yeast contribution to the observed phytase activity during bread leavening has sometimes been performed in conditions such as starvation in combination with 50°C. Harland et al. (1989) Effects of phytase from three yeasts on phytate reduction in Norwegian whole wheat flour. Cereal Chem. 4. 357-358.

The yeast phytase activity in conditions suitable for yeast growth were studied and it was observed that. provided absence of Pᵢ. *S*. *cerevisiae* is well adapted to utilize extracellular IP6 as a phosphorous source. The rate of IP6 degradation was rapid and supplied the yeast metabolic machinery with phosphorous without a decrease in growth rate. that arose from the increased energy cost of synthesizing the degrading enzymes. The phosphate-induced repression of phytase activity indicated that the *PHO* gene family in *S*. *cerevisiae* is involved.

In low concentrations of inorganic phosphate *S*. *cerevisiae* is known to induce the synthesis of at least three phosphatases that are exported out through the cellular membrane. It appears that the enzymes work best in oligomeric organization. as studied by Shnyreva et al. Shnyreva et al. (1996) Biochemical properties and excretion behavior of repressible acid phosphatases with altered subunit composition. Microbiol. Res. 151. 291-300, composed of a specific ratio between Pho5p (86%), Pho10p and Pho11p (14% together). The so-called "yeast phytase" may be the concerted action of the components in this oligomeric enzyme.

However, when using pNPP as substrate Shnyreva et al.. Shnyreva et al. (1996) Biochemica/ properties and excretion behavior ofrepressible acid phosphatases with ahered subunit composition. Microbiol. Res. 151. 291-300 have shown that also homopolymers of the individual secretory acid phosphatases have significant and different activities. In addition to being present in the largest amount. Pho5p was found to exert the highest specific activity. Fifteen times higher activity was obtained for Pho5p expressed alone as compared with Pho10p and Pho11p. respectively.

The *PHO5* deletion did not yield a less efficient strain with respect to IP6 degradation. Several explanations could fit the data: (i) *PHO5* was not important for phytase activity or (ii) the remaining level of Pho10p and Pho11p. organized without Pho5p. was sufficient for unchanged phytase activity. It may be that (iii) *PHO10* and *PHO11* became unregulated in the absence of *PHO5*. In fact. the absence of *PHO10* or *PHO11* has been shown to increase the Pho5p level Shnyreva et al. (1996) Biochemical properties and excretion behavior of repressible acid phosphatases with altered subunit composition. Microbiol. Res. 151. 291-300 and hence. the opposite may also be true. A (iv) different as yet unidentified phytase enzyme may exist. The *S*. *cerevisiae* gene *DIA3* for instance. encodes a protein largely related to Pho3. Pho5. Pho10 and Pho11 (see e.g. the YPD database). Dia3p may be extracellular.

Since the significance of the different secretory phosphatases in IP6 specifie degradation is not known a strain was created lacking only *PHO10* (YS10). Using strain YS10 it was demonstrated that *PHO10* was also dispensable for yeast phytase activity. shown by intact growth- and IP6 degradation rate. Thus, neither of *PHO3*, *PHO5* or *PHO10* was essential for high phytase activity. However, in a triple mutant (YM 10), a very small, but significant decrease in rate of IP6 degradation was observed in CBS and a pronounced decrease in YPD, demonstrating two things. First. Pho10p is an enzyme with phytase activity since a change was observed when *PHO10* was the only difference and second, a strain with only Pho11p alone (or an unknown phytase) still has a remarkable phytase activity in defined medium. For all these deletion strains the growth rate remained unchanged, so the net phytase activity is comparable also if expressed as biomass specific phytase activity. This further means that phosphorous never became limiting, even in a strain lacking three secretory phosphatases during growth on IP6. It is interesting that the main secretory phosphatase (Pho5p) is dispensable and that a strain encoding only one of two so-called minor secretory phosphatases. (Pho11p). is alone able to keep almost unchanged phytase activity.

To assess the phytase potential of solely Pho5p. two strains were constructed which expressed almost exclusively *PHO5* during growth in repressing medium. The data obtained using these strains show that *PHO5* by itself encodes a protein with phytase activity and is therefore likely to participate when a wildtype strain degrades IP6. When growing YM-p5 and YS-p5 in de-repressing media, additional phytase activity was observed as compared with the corresponding strains lacking the plasmids (FIG. 6). This was not necessarily expected since the increased rate of IP6 degradation leads to increase in Pᵢ concentration which in turn have a repressing impact on the genomic phytase encoding genes. The data reveals that yeast phytase activity is not the action of one gene product. However, simply by overexpressing one single gene strains were obtained with improved net phytase activity under both repressing and de-repressing conditions.

Furthermore, the regulatory components pho80p and pho85p are known to phosphorylate pho4p in conditions of high medium Pi levels. The phosphorylated pho4p retrieves affinity for a transporter and leaves the nucleus instead of acting as a transcription activator for several *PHO* genes, including the secretory phosphatases. If one or both of pho80p and pho85p is missing its kinase activity on pho4p is lost and pho4p will together with pho2p remain active as transcriptional activators for the secretory phosphatases. The resuming mutant constitutively expresses *PHO5*, *PHO10* and *PHO11.* Since deletions of *PHO80* or *PHO85* both yielded very strong constitutive phytase activity the involvement of the *PHO* system was again demonstrated.

It has previously been shown that the contribution of yeast to the phytase activity observed during bread leavening is very small, and not sufficient for a significant increase in iron and zinc absorption. Harland et al. (1989) Effects of phytase from three yeasts on phytate reduction in Norwegian whole wheat flour. Cereal Chem. 4. 357-358: Türk et al. (1992) Phytate degradation during bread making: effect of phytase addition. J. Cereal Science. 15. 281-294: Türk et al. (1996) Reduction in the levels of phytate during wholemeal bread making: Effect of yeast and wheat phytases. J. Cereal Science. 23. 257 - 264. The IP6 level and mineral uptake in human intestine was, however, improved when adding commercial *Aspergillus* phytase to dough or wheat rolls, respectively. Türk et al. (1992) Phytate degradation during bread making: effect of phytase addition. J. Cereal Science. 15. 281 - 294: Türk et al. (1996) Reduction in the levels of phytate during wholemeal bread making: Effect of yeast and wheat phytases. J. Cereal Science, 23, 257 - 264. it has been shown that the phytase activity is Pᵢ repressed. The concentration of Pᵢ was shown to be in the millimolar range which is above the level known to induce repression. Yoshida et al. (1989) Function of the PHO regulatory genes for repressible acid phosphatase systhesis in Saccharomyces cerevisiae, Mol. Gen. Genet. 217.40-46. (10 mM Pᵢ is often used as "high" and 0.2 mM as "low"; Ogawa et al. (2000) New components of a system for phosphate accumulation and polyphosphate metabolism in Saccharomyces cerevisiae revealed by genomic expression analysis. Mol. Biol. Cell. 11. 4309-4321). Hence, in the typical bread making situation the *PHO* genes encoding Pho5p. Pho10p and Pho11p are not very active and, as shown, phytase activity is not substantially expressed. Furthermore, it has been shown that the IP6 degradation in cereals must be extensive to improve the iron absorption, Brune et al. (1992) Human iron absorption from bread: Inhibiting effects of cereal fibre, phytate and inositol phosphates with different numbers of phosphate groups. J. Nutr. 122. 442-449. As little as 0.5 µmol/g dry sample of IP6 and IP5 is inhibitory. Sandberg et al. (1999) Inositol phosphates with different number of phosphate groups influence iron absorption in humans. Am. J. Clin. Nutr. 70. 240-246. The data on the content or IP6 in dough were expressed per wet weight (i.e. the dry content is higher), and even at the end of the fermentation it exceeds iron inhibitory levels. The reduction in IP6 taking place during dough fermentation with commercial baker's strains has been shown to be almost exclusively due to plant phytases present in the flour. A constitutively expressing yeast strain is desirable.

It has further been demonstrated herein an apparent preference for IP6 over pNPP when these were given together. In Nayaini et al.. Nayini et al. (1984) The phytase of yeast. Lebensm. Wiss. u-Technol. 17. 24-26. yeast phytase and phosphatase were purified from a total biomass extraction of a Baker's yeast cake. The distinction between phytase (which is a phosphalase, for which one or the substrates is phytate) and phosphatase activity was that a phosphatase was able to hydrolyze alpha-glycerophosphate but unable to hydrolyze IP6. The purified phytase was assessed for substrate specific activity using 11 different phosphorous sources. Phenyl-phosphate was one of these and the specific activity with this substrate was 40 times higher than for IP6. The data set forth above. shows virtually no degradation of pNPP until IP6 is depleted. Even at six times higher pNPP concentration the data indicate an inhibition of pNPP hydrolysis by IP6. Although the yeast phytase is not one single enzyme the activity was shown to be very precise in regard to the position on the inositol ring to be hydrolyzed, consistent with previous data on one commercial baker's strain. Türk et al. (2000) Inositol hexaphhosphate hydrolysis by baker's yeast. Capacity, kinetics, and degradation products. J. Agric. Food Chem. 48. 100-104. This specificity, the so-called 3-phytase activity, was true for all yeasts tested. including such different wild isolates of *S*. *cerevisiae* including those from fish intestine and clinical isolates of human pathogens, as well as for all depletion strains. Accordingly, the modified strains can be used to efficiently produce highly specific isomers of lower inositol phosphates.

In addition to the *Saccharomyces* phytase work we are screening non-*Saccharomyces* yeasts for the possibility of finding novel enzymes with desirable properties. Tested species are primarily from tropical- and cactus origin. Desired properties are high temperature optimum and/or a specificity yielding different isomers as compared with *S*. *cerevisiae.* So far, all tested species and strains have shown phytase activity. However, biochemical properties of the different enzymes have not yet been examined.

In addition to *S*. *cerevisiae*, other yeast species that are capable of expressing phytase can be transformed to exhibit increased phytase activity.

### 1. Primers for overexpression of PHO5

- *Forward primer:*
   5': CGGAATTCATGTTTAAATCTGTTGTTTATTC
- *Backwards primer:*
   3' : CGCTCGAGCTATTGTCTCAATAGACTGGC

Underlined sequences are EcoRI and Aval (yields end compatible to XhoI used to cut the pYX212 plasmid) restriction sites, respectively. The remaining sequences complement the beginning (from ATG) and end of *PHO5.*

**Sequence of the plasmid pYX212 with insert *PHO5.***

GAATTCATGTTTAAATCTGTTGTTTATTCAATTTTAGCCGCTTCTTTGGCCAATGC
AGGTACCATTCCCTTAGGCAAACTAGCCGATGTCGACAAGATTGGTACCCAAAAA
GATATCTTCCCATTTTGGGTGGTGCCGGACCATACTACTCTTTCCCTGGCGACTA
TGGTATTTCTCGTGATTTGCCTGAAGGTTGTGAAATGAAGCAACTGCAAATGGTT
GGTAGACATGGTGAAAGATACCCTACTGTCAGTCTGGCTAAGACTATCAAGAGTA
CATGGTATAAGTTGAGCAATTACACTCGTCAATTCAACGGCTCATTGTCATTCTTG
AACGATGATTACGAGTTTTTCATCCGTGATGACGATGATTTGGAAATGGAAACCA
CTTTTGCCAACTCGGACGATGTTTTGAACCCATACACTGGTGAAATGAACGCCAA
GAGACATGCTCGTGACTTCTTGGCTCAATACGGTTACATGGTCGAAAACCAAACC
AGTTTCGCCGTTTTTACCTCTAATTCTAAGAGATGTCATGACACTGCTCAATATTT
CATTGATGGTTTAGGTGACCAATTCAACATCACCTTGCAGACTGTCAGTGAAGCT
GAATCCGCTGGTGCCAACACTTTGAGTGCTTGTAACTCATGTCCTGCTTGGGACTA
CGATGCCAATGATGACATTGTAAATGAATACGACACAACCTACTTGGATGACATT
GCCAAGAGATTGAACAAGGAAAACAAGGGTTTGAACTTGACCTCAACTGACGCT
AGTACTTTATTCTCGTGGTGTGCATTTGAAGTGAACGCTAAAGGTTACAGTGATG
TCTGTGATATTTTCACCAAGGATGAATTAGTCCATTACTCCTACTACCAAGACTTG
CACACTTATTACCATGAGGGTCCAGGTTACGACATTATCAAGTCTGTCGGTTCCA
ACTTGTTCAATGCCTCAGTCAAATTATTAAAGCAAAGTGAGATTCAAGACCAAAA
GGTTTGGTTGAGTTTTACCCACGATACCGATATCCTAAACTTTTTGACCACCGCTG
GTATAATTGACGACAAAAACAACTTAACTGCCGAATACGTTCCATTCATGGGCAA
CACTTTCCACAGATCCTGGTACGTTCCTCAAGGTGCTCGTGTCTACACCGAAAAA
TTCCAATGTTCTAACGACACCTACGTCAGATACGTCATTAACGATGCTGTTGTTCC
AATTGAAACCTGTTCCACTGGTCCAGGGTTCTCTTGTGAAATCAATGACTTCTACG
ACTATGCTGAAAAGAGAGTAGCCGGTACTGACTTCCTAAAGGTCTGTAACGTCAG
CAGCGTCAGTAACTCTACTGAATTGACCTTCTACTGGGACTGGAACACTACTCAT
TACAACGCCAGTCTATTGAGACAATAGCCCGGGTATCCGTATGATGTGCCTGACT
ACGCATGATATCTCGAGCTCAGCTAGCTAACTGAATAAGGAACAATGAACGTTTT
TCCTTTCTCTTGTTCCTAGTATTAATGACTGACCGATACATCCCTTTTTTTTTTTGT
CTTTGTCTAGCTCCAGCTTTTGTTCCCTTTAGTGAGGGTTAATTCAATTCACTGGC
CGTCGTTTTACAACGTCGTGACTGGGAAAACCCTGGCGTTACCCAACTTAATCGC

CTTGCAGCACATCCCCCTTTCGCCAGCTGGCGTAATAGCGAAGAGGCCCGCACCG
ATCGCCCTTCCCAACAGTTGCGCAGCCTGAATGGCGAATGGCGCGACGCGCCCTG
TAGCGGCGCATTAAGCGCGGCGGGTGTGGTGGTTACGCGCAGCGTGACCGCTAC
ACTTGCCAGCGCCCTAGCGCCCGCTCCTTTCGCTTTCTTCCCTTCCTTTCTCGCCAC
GTTCGCCGGCTTTCCCCGTCAAGCTCTAAATCGGGGGCTCCCTTTAGGGTTCCGAT
TTAGTGGTTTACGGCACCTCGACCCCAAAAAACTTGATTAGGGTGATGGTTCACG
TAGTGGGCCATCGCCCTGATAGACGGTTTTTCGCCCTTTGACGTTGGAGTCCACGT
TCTTTAATAGTGGACTCTTGTTCCAAACTGGAACAACACTCAACCCTATCTCGGTC
TATTCTTTTGATTTATAAGGGATTTTGCCGATTTCGGCCTATTGGTTAAAAAATGA
GCTGATTTAACAAAAATTTAACGCGAATTTTAACAAAATATTAACGTTTACAATT
TCCTGATGCGGTATTTTCTCCTTACGCATCTGTGCGGTATTTCACACCGCATAGGG
TAATAACTGATATAATTAAATTGAAGCTCTAATTTGTGAGTTTAGTATACATGCAT
TTACTTATAATACAGTTTTTTAGTTTTGCTGGCCGCATCTTCTCAAATATGCTTCCC
AGCCTGCTTTTCTGTAACGTTCACCCTGTACCTTAGCATCCCTTCCCTTTGCAAAT
AGTCCTCTTCCAACAATAATAATGTCAGATCCTGTAGAGACCACATCATCCACGG
TTCTATACTGTTGACCCAATGCGTCTCCCTTGTCATCTAAACCCACACCGGGTGTC
ATAATCAACCAATCGTAACCTTCATCTCTTCCACCCATGTCTCTTTGAGCAATAAA
GCCGATAACAAAATCTTTGTCGCTCTTCGCAATGTCAACAGTACCCTTAGTATATT
CTCCAGTAGATAGGGAGCCCTTGCATGACAATTCTGCTAACATCAAAAGGCCTCT
AGGTTCCTTTGTTACTTCTTCTGCCGCCTGCTTCAAACCGCTAACAATACCTGGCC
CCAGCACACCGTGTGCATTCGTAATGTCTGCCCATTCTGCTATTCTGTATACACCC
GCAGAGTACTGCAATTTGACTGTATTACCAATGTCAGCAAATTTTCTGTCTTCGAA
GAGTAAAAAATTGTACTTGGCGGATAATGCCTTTAGCGGCTTAACTGTGCCCTCC
ATCGAAAAATCAGTCAATATATCCACATGTGTTTTTAGTAAACAAATTTTGGGAC
CTAATGCTTCAACTAACTCCAGTAATTCCTTGGTGGTACGAACATCCAATGAAGC
ACACAAGTTTGTTTGCTTTTCGTGCATGATATTAAATAGCTTGGCAGCAACAGGA
CTAGGATGAGTAGCAGCACGTTCCTTATATGTAGCTTTCGACATGATTTATCTTCG
TTTCCTGCAGGTTTTTGTTCTGTGCAGTTGGGTTAAGAATACTGGGCAATTTCATG
TTTCTTCAACACTACATATGCGTATATATACCAATCTAAGTCTGTGCTCCTTCCTT
CGTTCTTCCTTCTGTTCGGAGATTACCGAATCAAAAAAATTTCAAAGAAACCGAA
ATCAAAAAAAAGAATAAAAAAAAAATGATGAATTGAATTGAAAAGCTGTGGTAT
GGTGCACTCTCAGTACAATCTGCTCTGATGCCGCATAGTTAAGCCAGCCCCGACA
CCCGCCAACACCCGCTGACGCGCCCTGACGGGCTTGTCTGCTCCCGGCATCCGCT
TACAGACAAGCTGTGACCGTCTCCGGGAGCTGCATGTGTCAGAGGTTTTCACCGT
CATCACCGAAACGCGCGAGACGAAAGGGCCTCGTGATACGCCTATTTTTATAGGT
TAATGTCATGATAATAATGGTTTCTTAGACGTGCGGCCGCTCTAGAACTAGTGGA
TCAATTCCACGGACTATAGACTATACTAGTATACTCCGTCTACTGTACGATACACT
TCCGCTCAGGTCCTTGTCCTTTAACGAGGCCTTACCACTCTTTTGTTACTCTATTGA
TCCAGCTCAGCAAAGGCAGTGTGATCTAAGATTCTATCTTCGCGATGTAGTAAAA
CTAGCTAGACCGAGAAAGAGACTAGAAATGCAAAAGGCACTTCTACAATGGCTG
CCATCATTATTATCCGATGTGACGCTGCAGCTTCTCAATGATATTCGAATACGCTT
TGAGGAGATACAGCCTAATATCCGACAAACTGTTTTACAGATTTACGATCGTACT
TGTTACCCATCATTGAATTTTGAACATCCGAACCTGGGAGTTTTCCCTGAAACAG
ATAGTATATTTGAACCTGTATAATAATATATAGTCTAGCGCTTTACGGAAGACAA
TGTATGTATTTCGGTTCCTGGAGAAACTATTGCATCTATTGCATAGGTAATCTTGC
ACGTCGCATCCCCGGTTCATTTTCTGCGTTTCCATCTTGCACTTCAATAGCATATC
TTTGTTAACGAAGCATCTGTGCTTCATTTTGTAGAACAAAAATGCAACGCGAGAG
CGCTAATTTTTCAAACAAAGAATCTGAGCTGCATTTTTACAGAACAGAAATGCAA
CGCGAAAGCGCTATTTTACCAACGAAGAATCTGTGCTTCATTTTTGTAAAACAAA
AATGCAACGCGAGAGCGCTAATTTTTCAAACAAAGAATCTGAGCTGCATTTTTAC

AGAACAGAAATGCAACGCGAGAGCGCTATTTTACCAACAAAGAATCTATACTTCT
TTTTTGTTCTACAAAAATGCATCCCGAGAGCGCTATTTTTCTAACAAAGCATCTTA
GATTACTTTTTTTCTCCTTTGTGCGCTCTATAATGCAGTCTCTTGATAACTTTTTGC
ACTGTAGGTCCGTTAAGGTTAGAAGAAGGCTACTTTGGTGTCTATTTTCTCTTCCA
TAAAAAAAGCCTGACTCCACTTCCCGCGTTTACTGATTACTAGCGAAGCTGCGGG
TGCATTTTTTCAAGATAAAGGCATCCCCGATTATATTCTATACCGATGTGGATTGC
GCATACTTTGTGAACAGAAAGTGATAGCGTTGATGATTCTTCATTGGTCAGAAAA
TTATGAACGGTTTCTTCTATTTTGTCTCTATATACTACGTATAGGAAATGTTTACA
TTTTCGTATTGTTTTCGATTCACTCTATGAATAGTTCTTACTACAATTTTTTTGTCT
AAAGAGTAATACTAGAGATAAACATAAAAAATGTAGAGGTCGAGTTTAGATGCA
AGTTCAAGGAGCGAAAGGTGGATGGGTAGGTTATATAGGGATATAGCACAGAGA
TATATAGCAAAGAGATACTTTTGAGCAATGTTTGTGGAAGCGGTATTCGCAATAT
TTTAGTAGCTCGTTACAGTCCGGTGCGTTTTTGGTTTTTTGAAAGTGCGTCTTCAG
AGCGCTTTTGGTTTTCAAAAGCGCTCTGAAGTTCCTATACTTTCTAGAGAATAGGA
ACTTCGGAATAGGAACTTCAAAGCGTTTCCGAAAACGAGCGCTTCCGAAAATGCA
ACGCGAGCTGCGCACATACAGCTCACTGTTCACGTCGCACCTATATCTGCGTGTT
GCCTGTATATATATATACATGAGAAGAACGGCATAGTGCGTGTTTATGCTTAAAT
GCGTACTTATATGCGTCTATTTATGTAGGATGAAAGGTAGTCTAGTACCTCCTGTG
ATATTATCCCATTCCATGCGGGGTATCGTATGCTTCCTTCAGCACTACCCTTTAGC
TGTTCTATATGCTGCCACTCCTCAATTGGATTAGTCTCATCCTTCAATGCTATCATT
TCCTTTGATATTGGATCATATGCATAGTACCGAGAAACTAGTGCGAAGTAGTGAT
CAGGTATTGCTGTTATCTGATGAGTATACGTTGTCCTGGCCACGGCAGAAGCACG
CTTATCGCTCCAATTTCCCACAACATTAGTCAACTCCGTTAGGCCCTTCATTGAAA
GAAATGAGGTCATCAAATGTCTTCCAATGTGAGATTTTGGGCCATTTTTTATAGCA
AAGATTGAATAAGGCGCATTTTTCTTCAAAGCTGCGGCCGCACTCTCACTAGTAC
GTCAGGTGGCACTTTTCGGGGAAATGTGCGCGGAACCCCTATTTGTTTATTTTTCT
AAATACATTCAAATATGTATCCGCTCATGAGACAATAACCGTGATAAATGCTTCA
ATAATATTGAAAAAGGAAGAGTATGAGTATTCAACATTTCCGTGTCGCCCTTATT
CCCTTTTTTGCGGCATTTTGCCTTCCTGTTTTTGCTCACCCAGAAACGCTGGTGAA
AGTAAAAGATGCTGAAGATCAGTTGGGTGCACGAGTGGGTTACATCGAACTGGA
TCTCAACAGCGGTAAGATCCTTGAGAGTTTTCGCCCCGAAGAACGTTTTCCAATG
ATGAGCACTTTTAAAGTTCTGCTATGTGGCGCGGTATTATCCCGTATTGACGCCGG
GCAAGAGCAACTCGCTCGCCGCATACACTATTCTCAGAATGACTTGGTTGAGTAC
TCACCAGTCACAGAAAAGCATCTTACGGATGGCATGACAGTAAGAGAATTATGC
AGTGCTGCCATAACCATGAGTGATAACACTGCGGCCAACTTACTTCTGACAACGA
TCGGAGGACCGAAGGAGCTAACCGCTTTTTTGGACAACATGGGGGATCATGTAAC
TCGCCTTGATCGTTGGGAACCGGAGCTGAATGAAGCCATACCAAACGACGAGCG
TGACACCACGATGCCTGTAGCAATGGCAACAACGTTGCGCAAACTATTAACTGGC
GAACTACTTACTCTAGCTTCCCGGCAACAATTAATAGACTGGATGGAGGCGGATA
AAGTTGCAGGACCACTTCTGCGCTCGGCCCTTCCGGCTGGCTGGTTTATTGCTGAT
AAATCTGGAGCCGGTGAGCGTGGGTCTCGCGGTATCATTGCAGCACTGGGGCCAG
ATGGTAAGCCCTCCCGTATCGTAGTTATCTACACGACGGGGAGTCAGGCAACTAT
GGATGAACGAAATAGACAGATCGCTGAGATAGGTGCCTCACTGATTAAGCATTG
GTAACTGTCAGACCAAGTTTACTCATATATACTTTAGATTGATTTAAAACTTCATT
TTTAATTTAAAAGGATCTAGGTGAAGATCCTTTTTGATAATCTCATGACCAAAATC
CCTTAACGTGAGTTTTCGTTCCACTGAGCGTCAGACCCCGTAGAAAAGATCAAAG
GATCTTCTTGAGATCCTTTTTTTCTGCGCGTAATCTGCTGCTTGCAAACAAAAAAA
CCACCGCTACCAGCGGTGGTTTGTTTGCCGGATCAAGAGCTACCAACTCTTTTTCC
GAAGGTAACTGGCTTCAGCAGAGCGCAGATACCAAATACTGTCCTTCTAGTGTAG
CCGTAGTTAGGCCACCACTTCAAGAACTCTGTAGCACCGCCTACATACCTCGCTC

TGCTAATCCTGTTACCAGTGGCTGCTGCCAGTGGCGATAAGTCGTGTCTTACCGG
GTTGGACTCAAGACGATAGTTACCGGATAAGGCGCAGCGGTCGGGCTGAACGGG
GGGTTCGTGCACACAGCCCAGCTTGGAGCGAACGACCTACACCGAACTGAGATA
CCTACAGCGTGAGCTATGAGAAAGCGCCACGCTTCCCGAAGGGAGAAAGGCGGA
CAGGTATCCGGTAAGCGGCAGGGTCGGAACAGGAGAGCGCACGAGGGAGCTTCC
AGGGGGAAACGCCTGGTATCTTTATAGTCCTGTCGGGTTTCGCCACCTCTGACTTG
AGCGTCGATTTTTGTGATGCTCGTCAGGGGGGCGGAGCCTATGGAAAAACGCCAG
CAACGCGGCCTTTTTACGGTTCCTGGCCTTTTGCTGGCCTTTTGCTCACATGTTCTT
TCCTGCGTTATCCCCTGATTCTGTGGATAACCGTATTACCGCCTTTGAGTGAGCTG
ATACCGGTCGCCGCAGCCGAACGACCGAGCGCAGCGAGTCAGTGAGCGAGGAAG
CGGAAGAGCGCCCAATACGCAAACCGCCTCTCCCCGCGCGTTGGCCGATTCATTA
ATGCAGCTGGCACGACAGGTTTCCCGACTGGAAAGCGGGCAGTGAGCGCAACGC
AATTAATGTGAGTTACCTCACTCATTAGGCACCCCAGGCTTTACACTTTATGCTTC
CGGCTCCTATGTTGTGTGGAATTGTGAGCGGATAACAATTTCACACAGGAAACAG
CTATGACCATGATTACGCCAAGCTCGAAATACGACTCACTATAGGGCGAATTGGG
TACCGGGCCGGCCGTCGAGCTTGATGGCATCGTGGTGTCACGCTCGTCGTTTGGT
ATGGCTTCATTCAGCTCCGGTTCCCAACGATCAAGGCGAGTTACATGATCCCCCA
TGTTGTGAAAAAAGCGGTTAGCTCTTCGGTCCTCCGATCGTTGTCAGAAGTAAG
TTGGCCGCAGTGTTATCACTCATGGTTATGGCAGGAACTGCATAATTCTCTTACTG
TCATGCCATCCGTAAGATGCTTTTCTGTGACTGGTGTACTCAACCAAGTCATTCTG
AGAATAGTGTATGCGGCGACCGAGTTGCTCTTGCCCGGCGTCAACACGGGATAAT
ACCGCGCCACATAGCAGAACTTTAAAAGTGCTCATCATTGGAAAACGTTCTTCGG
GGCGAAAACTCTCAAGGATCTTACCGCTGTTGAGATCCAGTTCGATGTAACCCAC
TCGTGCACCCAACTGATCTTCAGCATCTTTTACTTTCACCAGCGTTTCTGGGTGAG
CAAAAACAGGAAGGCAAAATGCCGCAAAAAAGGGAATAAGGGCGACACGGAAA
TGTTGAATACTCATACTCTTCCTTTTTCAATATTATTGAAGCATTTATCAGGGTTAT
TGTCTCATGAGCGATACATATTTGAATGTATTTAGAAAAATAAACAAATAGGGGT
TCCGCGCACATTTCCCCGAAAAGTGCCACCTGACGTCTAAGAAACCATTATTATC
ATGACATTAACCTATAAAAATAGGCGTATCACGAGGCCCTTTCGTCTTCAAGAAT
TGGGGATCTACGTATGGTCATTCTTCTTCAGATTCCCTCATGGAGAAGTGCGGCA
GATGTATATGACAGAGTCGCCAGTTTCCAAGAGACTTTATTCAGGCACTTCCATG
ATAGGCAAGAGAGAAGACCCAGAGATGTTGTTGTCCTAGTTACACATGGTATTTA
TTCCAGAGTATTCCTGATGAAATGGTTTAGATGGACATACGAAGAGTTTGAATCG
TTTACCAATGTTCCTAACGGGAGCGTAATGGTGATGGAACTGGACGAATCCATCA
ATAGATACGTCCTGAGGACCGTGCTACCCAAATGGACTGATTGTGAGGGAGACCT
AACTACATAGTGTTTAAAGATTACGGATATTTAACTTACTTAGAATAATGCCATTT
TTTTGAGTTATAATAATCCTACGTTAGTGTGAGCGGGATTTAAACTGTGAGGACC
TCAATACATTCAGACACTTCTGACGGTATCACCCTACTTATTCCCTTCGAGATTAT
ATCTAGGAACCCATCAGGTTGGTGGAAGATTACCCGTTCTAAGACTTTTCAGCTT
CCTCTATTGATGTTACACTCGGACACCCCTTTTCTGGCATCCAGTTTTTAATCTTCA
GTGGCATGTGAGATTCTCCGAAATTAATTAAAGCAATCACACAATTCTCTCGGAT
ACCACCTCGGTTGAAACTGACAGGTGGTTTGTTACGCATGCTAATGCAAAGGAGC
CTATATACCTTTGGCTCGGCTGCTGTAACAGGGAATATAAAGGGCAGCATAATTT
AGGAGTTTAGTGAACTTGCAACATTTACTATTTTCCCTTCTTACGTAAATATTTTT
CTTTTTAATTCTAAATCAATCTTTTTCAATTTTTTGTTTGTATTCTTTTCTTGCTTAA
ATCTATAACTACAAAAAACACATACAGG

Underlined sequences show sites for primers
Blue nucleotides represent the inserted *PHO5*

### 2. Primers for deletion of PHO10

5': CGATAGATTCAAGCTCAGTTTCGCCTTGGTTGTAAAGTAGGCAGCTGAAGCTT
CGTACGC-3'

5': GGTCTATTTACTGTTTTAATAAAGTGTCGTTGTAGTGCTTGGGGCAGATGATG
TCGAGGCG-3'.

These oligonucleotides were used as primers for construction of deletion cassettes. Underlined sequences are complementary to *HIS5* in the template, and non-underlined parts to flanking regions of *PHO10.* The *PHO10* deletions were generated by PCR-mediated gene replacement using *HIS5* from *Saccharomyces pombe* (corresponding to *HIS3* in *S*. *cerevisiae*) as the selectable marker. As template DNA the plasmid pFA6a-HIS3MX6 was used.

### Resulting PCR product used for deletion of PHO10 by homologous recombination

CGATAGATTCAAGCTCAGTTTCGCCTTGGTTGTAAAGTAGCCAGCTGAAGCTTCGTAC
GCTGCAGGTCGACGGATCCCCGGGTTAATTAAGGCGCGCCAGATCTGTTTAGCTT
GCCTCGTCCCCGCCGGGTCACCCGGCCAGCGACATGGAGGCCCAGAATACCCTCC
TTGACAGTCTTGACGTGCGCAGCTCAGGGGCATGATGTGACTGTCGCCCGTACAT
TTAGCCCATACATCCCCATGTATAATCATTTGCATCCATACATTTTGATGGCCGCA
CGGCGCGAAGCAAAAATTACGGCTCCTCGCTGCAGACCTGCGAGCAGGGAAACG
CTCCCCTCACAGACGCGTTGAATTGTCCCCACGCCGCGCCCCTGTGAGAAATATA
AAAGGTTAGGATTTGCCACTGAGGTTCTTCTTTCATATACTTCCTTTTAAAATCTTG
CTAGGATACAGTTCTCACATCACATCCGAACATAAACAACCATGGGTAGGAGGG
CTTTTGTAGAAAGAAATACGAACGAAACGAAAATCAGCGTTGCCATCGCTTTGGA
CAAAGCTCCCTTACCTGAAGAGTCGAATTTTATTGATGAACTTATAACTTCCAAG
CATGCAAACCAAAAGGGAGAACAAGTAATCCAAGTAGACACGGGAATTGGATTC
TTGGATCACATGTATCATGCACTGGCTAAACATGCAGGCTGGAGCTTACGACTTT
ACTCAAGAGGTGATTTAATCATCGATGATCATCACACTGCAGAAGATACTGCTAT
TGCACTTGGTATTGCATTCAAGCAGGCTATGGGTAACTTTGCCGGCGTTAAAAGA
TTTGGACATGCTTATTGTCCACTTGACGAAGCTCTTTCTAGAAGCGTAGTTGACTT
GTCGGGACGGCCCTATGCTGTTATCGATTTGGGATTAAAGCGTGAAAAGGTTGGG
GAATTGTCCTGTGAAATGATCCCTCACTTACTATATTCCTTTTCGGTAGCAGCTGG
AATTACTTTGCATGTTACCTGCTTATATGGTAGTAATGACCATCATCGTGCTGAAA
GCGCTTTTAAATCTCTGGCTGTTGCCATGCGCGCGGCTACTAGTCTTACTGGAAGT
TCTGAAGTCCCAAGCACGAAGGGAGTGTTGTAAAGAGTACTGACAATAAAAAGA
TTCTTGTTTTCAAGAACTTGTCATTTGTATAGTTTTTTTATATTGTAGTTGTTCTAT
TTTAATCAAATGTTAGCGTGATTTATATTTTTTTTCGCCTCGACATCATCTGCCCCA
AGCACTACAACGACACTTTATTAAAACAGTAAATAGACC

Underlined sequences represent primers (or complementary strand to primer) used Italic shows sequence corresponding to *PHO10* (SGD)
Blue sequence show *HIS5* (*S. pombe;* corresponding to *HIS3 S. cerevisiae*) used as selectable marker.

### 3. Primers for overexpression of PHO4

- *Forward primer (JP42)*
   5'-CGGAATTCATGGGCCGTACAACTTCTGAGG-3'
- *Backwards primers (JT42)*
   5'-CGCTCGAGTCACGTGCTCACGTTCTGCAG-3'

Underlined sequences complement EcoRI and XhoI restriction sites in pYX212 and the rest are sequences that complement start and end regions in *PHO4* (SGD).

**Sequence of pYX212 containing the insert *PHO4***

GAATTCATGGGCCGTACAACTTCTGAGGGAATACACGGTTTTGTGGACGATCTAG
AGCCCAAGAGCAGCATTCTTGATAAAGTCGGAGACTTTATCACCGTAAACACGAA
ACGGCATGATGGGCGCGAGGACTTCAACGAGCAAAACGACGAGCTGAACAGTCA
AGAGAACCACAACAGCAGTGAGAATGGGAACGAGAATGAAAATGAACAAGACA
GTCTCGCGTTGGACGACCTAGACCGCGCCTTTGAGCTGGTGGAAGGTATGGATAT
GGACTGGATGATGCCCTCGCATGCGCACCACTCCCCAGCTACAACTGCTACAATC
AAGCCGCGGCTATTATATTCGCCGCTAATACACACGCAAAGTGCGGTTCCCGTAA
CCATTTCGCCGAACTTGGTCGCTACTGCTACTTCCACCACATCCGCTAACAAAGTC
ACTAAAAACAAGAGTAATAGTAGTCCGTATTTGAACAAGCGCAGAGGTAAACCC
GGGCCGGATTCGGCCACTTCGCTGTTCGAATTGCCCGACAGCGTTATCCCAACTC
CGAAACCGAAACCGAAACCAAAGCAATATCCGAAAGTTATTCTGCCGTCGAACA
GCACAAGACGCGTATCACCGGTCACGGCCAAGACCAGCAGCAGCGCAGAAGGCG
TGGTCGTAGCAAGTGAGTCTCCTGTAATCGCGCCGCACGGATCGAGCCATTCGCG
GTCGCTGAGTAAGCGACGGTCATCGGGCGCGCTCGTGGACGATGACAAGCGCGA
ATCACACAAGCATGCAGAGCAAGCACGGCGTAATCGATTAGCGGTCGCGCTGCA
CGAACTGGCGTCTTTAATCCCCGCGGAGTGGAAACAGCAAAATGTGTCGGCCGCG
CCGTCCAAAGCGACCACCGTGGAGGCGGCCTGCCGGTACATCCGTCACCTACAGC
AGAACGTGAGCACGTGACTCGAGCTCAGCTAGCTAACTGAATAAGGAACAATGA
ACGTTTTCCTTTCTCTTGTTCCTAGTATTAATGACTGACCGATACATCCCTTTTTT
TTTTTGTCTTTGTCTAGCTCCAGCTTTTGTTCCCTTTAGTGAGGGTTAATTCAATTC
ACTGGCCGTCGTTTTACAACGTCGTGACTGGGAAAACCCTGGCGTTACCCAACTT
AATCGCCTTGCAGCACATCCCCCTTTCGCCAGCTGGCGTAATAGCGAAGAGGCCC
GCACCGATCGCCCTTCCCAACAGTTGCGCAGCCTGAATGGCGAATGGCGCGACGC
GCCCTGTAGCGGCGCATTAAGCGCGGCGGGTGTGGTGGTTACGCGCAGCGTGACC
GCTACACTTGCCAGCGCCCTAGCGCCCGCTCCTTTCGCTTTCTTCCCTTCCTTTCTC
GCCACGTTCGCCGGCTTTCCCCGTCAAGCTCTAAATCGGGGGCTCCCTTTAGGGTT
CCGATTTAGTGGTTTACGGCACCTCGACCCCAAAAAACTTGATTAGGGTGATGGT
TCACGTAGTGGGCCATCGCCCTGATAGACGGTTTTTCGCCCTTTGACGTTGGAGTC
CACGTTCTTTAATAGTGGACTCTTGTTCCAAACTGGAACAACACTCAACCCTATCT
CGGTCTATTCTTTTGATTTATAAGGGATTTTGCCGATTTCGGCCTATTGGTTAAAA
AATGAGCTGATTTAACAAAAATTTAACGCGAATTTTAACAAAATATTAACGTTTA
CAATTTCCTGATGCGGTATTTTCTCCTTACGCATCTGTGCGGTATTTCACACCGCA
TAGGGTAATAACTGATATAATTAAATTGAAGCTCTAATTTGTGAGTTTAGTATAC
ATGCATTTACTTATAATACAGTTTTTTAGTTTTGCTGGCCGCATCTTCTCAAATAT
GCTTCCCAGCCTGCTTTTCTGTAACGTTCACCCTGTACCTTAGCATCCCTTCCCTTT

GCAAATAGTCCTCTTCCAACAATAATAATGTCAGATCCTGTAGAGACCACATCAT
CCACGGTTCTATACTGTTGACCCAATGCGTCTCCCTTGTCATCTAAACCCACACCG
GGTGTCATAATCAACCAATCGTAACCTTCATCTCTTCCACCCATGTCTCTTTGAGC
AATAAAGCCGATAACAAAATCTTTGTCGCTCTTCGCAATGTCAACAGTACCCTTA
GTATATTCTCCAGTAGATAGGGAGCCCTTGCATGACAATTCTGCTAACATCAAAA
GGCCTCTAGGTTCCTTTGTTACTTCTTCTGCCGCCTGCTTCAAACCGCTAACAATA
CCTGGCCCCAGCACACCGTGTGCATTCGTAATGTCTGCCCATTCTGCTATTCTGTA
TACACCCGCAGAGTACTGCAATTTGACTGTATTACCAATGTCAGCAAATTTTCTGT
CTTCGAAGAGTAAAAAATTGTACTTGGCGGATAATGCCTTTAGCGGCTTAACTGT
GCCCTCCATCGAAAAATCAGTCAATATATCCACATGTGTTTTTAGTAAACAAATTT
TGGGACCTAATGCTTCAACTAACTCCAGTAATTCCTTGGTGGTACGAACATCCAA
TGAAGCACACAAGTTTGTTTGCTTTTCGTGCATGATATTAAATAGCTTGGCAGCA
ACAGGACTAGGATGAGTAGCAGCACGTTCCTTATATGTAGCTTTCGACATGATTT
ATCTTCGTTTCCTGCAGGTTTTTGTTCTGTGCAGTTGGGTTAAGAATACTGGGCAA
TTTCATGTTTCTTCAACACTACATATGCGTATATATACCAATCTAAGTCTGTGCTC
CTTCCTTCGTTCTTCCTTCTGTTCGGAGATTACCGAATCAAAAAAATTTCAAAGAA
ACCGAAATCAAAAAAAAGAATAAAAAAAAAATGATGAATTGAATTGAAAAGCTG
TGGTATGGTGCACTCTCAGTACAATCTGCTCTGATGCCGCATAGTTAAGCCAGCC
CCGACACCCGCCAACACCCGCTGACGCGCCCTGACGGGCTTGTCTGCTCCCGGCA
TCCGCTTACAGACAAGCTGTGACCGTCTCCGGGAGCTGCATGTGTCAGAGGTTTT
CACCGTCATCACCGAAACGCGCGAGACGAAAGGGCCTCGTGATACGCCTATTTTT
ATAGGTTAATGTCATGATAATAATGGTTTCTTAGACGTGCGGCCGCTCTAGAACT
AGTGGATCAATTCCACGGACTATAGACTATACTAGTATACTCCGTCTACTGTACG
ATACACTTCCGCTCAGGTCCTTGTCCTTTAACGAGGCCTTACCACTCTTTTGTTAC
TCTATTGATCCAGCTCAGCAAAGGCAGTGTGATCTAAGATTCTATCTTCGCGATGT
AGTAAAACTAGCTAGACCGAGAAAGAGACTAGAAATGCAAAAGGCACTTCTACA
ATGGCTGCCATCATTATTATCCGATGTGACGCTGCAGCTTCTCAATGATATTCGAA
TACGCTTTGAGGAGATACAGCCTAATATCCGACAAACTGTTTTACAGATTTACGA
TCGTACTTGTTACCCATCATTGAATTTTGAACATCCGAACCTGGGAGTTTTCCCTG
AAACAGATAGTATATTTGAACCTGTATAATAATATATAGTCTAGCGCTTTACGGA
AGACAATGTATGTATTTCGGTTCCTGGAGAAACTATTGCATCTATTGCATAGGTA
ATCTTGCACGTCGCATCCCCGGTTCATTTTCTGCGTTTCCATCTTGCACTTCAATA
GCATATCTTTGTTAACGAAGCATCTGTGCTTCATTTTGTAGAACAAAAATGCAAC
GCGAGAGCGCTAATTTTTCAAACAAAGAATCTGAGCTGCATTTTTACAGAACAGA
AATGCAACGCGAAAGCGCTATTTTACCAACGAAGAATCTGTGCTTCATTTTTGTA
AAACAAAAATGCAACGCGAGAGCGCTAATTTTTCAAACAAAGAATCTGAGCTGC
ATTTTTACAGAACAGAAATGCAACGCGAGAGCGCTATTTTACCAACAAAGAATCT
ATACTTCTTTTTTGTTCTACAAAAATGCATCCCGAGAGCGCTATTTTTCTAACAAA
GCATCTTAGATTACTTTTTTTCTCCTTTGTGCGCTCTATAATGCAGTCTCTTGATAA
CTTTTTGCACTGTAGGTCCGTTAAGGTTAGAAGAAGGCTACTTTGGTGTCTATTTT
CTCTTCCATAAAAAAAGCCTGACTCCACTTCCCGCGTTTACTGATTACTAGCGAA
GCTGCGGGTGCATTTTTTCAAGATAAAGGCATCCCCGATTATATTCTATACCGATG
TGGATTGCGCATACTTTGTGAACAGAAAGTGATAGCGTTGATGATTCTTCATTGG
TCAGAAAATTATGAACGGTTTCTTCTATTTTGTCTCTATATACTACGTATAGGAAA
TGTTTACATTTTCGTATTGTTTTCGATTCACTCTATGAATAGTTCTTACTACAATTT
TTTTGTCTAAAGAGTAATACTAGAGATAAACATAAAAAATGTAGAGGTCGAGTTT
AGATGCAAGTTCAAGGAGCGAAAGGTGGATGGGTAGGTTATATAGGGATATAGC
ACAGAGATATATAGCAAAGAGATACTTTTGAGCAATGTTTGTGGAAGCGGTATTC
GCAATATTTTAGTAGCTCGTTACAGTCCGGTGCGTTTTTGGTTTTTTGAAAGTGCG
TCTTCAGAGCGCTTTTGGTTTTCAAAAGCGCTCTGAAGTTCCTATACTTTCTAGAG

AATAGGAACTTCGGAATAGGAACTTCAAAGCGTTTCCGAAAACGAGCGCTTCCG
AAAATGCAACGCGAGCTGCGCACATACAGCTCACTGTTCACGTCGCACCTATATC
TGCGTGTTGCCTGTATATATATATACATGAGAAGAACGGCATAGTGCGTGTTTAT
GCTTAAATGCGTACTTATATGCGTCTATTTATGTAGGATGAAAGGTAGTCTAGTA
CCTCCTGTGATATTATCCCATTCCATGCGGGGTATCGTATGCTTCCTTCAGCACTA
CCCTTTAGCTGTTCTATATGCTGCCACTCCTCAATTGGATTAGTCTCATCCTTCAAT
GCTATCATTTCCTTTGATATTGGATCATATGCATAGTACCGAGAAACTAGTGCGA
AGTAGTGATCAGGTATTGCTGTTATCTGATGAGTATACGTTGTCCTGGCCACGGC
AGAAGCACGCTTATCGCTCCAATTTCCCACAACATTAGTCAACTCCGTTAGGCCC
TTCATTGAAAGAAATGAGGTCATCAAATGTCTTCCAATGTGAGATTTTGGGCCAT
TTTTTATAGCAAAGATTGAATAAGGCGCATTTTTCTTCAAAGCTGCGGCCGCACTC
TCACTAGTACGTCAGGTGGCACTTTTCGGGGAAATGTGCGCGGAACCCCTATTTG
TTTATTTTTCTAAATACATTCAAATATGTATCCGCTCATGAGACAATAACCGTGAT
AAATGCTTCAATAATATTGAAAAAGGAAGAGTATGAGTATTCAACATTTCCGTGT
CGCCCTTATTCCCTTTTTTGCGGCATTTTGCCTTCCTGTTTTTGCTCACCCAGAAAC
GCTGGTGAAAGTAAAAGATGCTGAAGATCAGTTGGGTGCACGAGTGGGTTACAT
CGAACTGGATCTCAACAGCGGTAAGATCCTTGAGAGTTTTCGCCCCGAAGAACGT
TTTCCAATGATGAGCACTTTTAAAGTTCTGCTATGTGGCGCGGTATTATCCCGTAT
TGACGCCGGGCAAGAGCAACTCGGTCGCCGCATACACTATTCTCAGAATGACTTG
GTTGAGTACTCACCAGTCACAGAAAAGCATCTTACGGATGGCATGACAGTAAGA
GAATTATGCAGTGCTGCCATAACCATGAGTGATAACACTGCGGCCAACTTACTTC
TGACAACGATCGGAGGACCGAAGGAGCTAACCGCTTTTTTGGACAACATGGGGG
ATCATGTAACTCGCCTTGATCGTTGGGAACCGGAGCTGAATGAAGCCATACCAAA
CGACGAGCGTGACACCACGATGCCTGTAGCAATGGCAACAACGTTGCGCAAACT
ATTAACTGGCGAACTACTTACTCTAGCTTCCCGGCAACAATTAATAGACTGGATG
GAGGCGGATAAAGTTGCAGGACCACTTCTGCGCTCGGCCCTTCCGGCTGGCTGGT
TTATTGCTGATAAATCTGGAGCCGGTGAGCGTGGGTCTCGCGGTATCATTGCAGC
ACTGGGGCCAGATGGTAAGCCCTCCCGTATCGTAGTTATCTACACGACGGGGAGT
CAGGCAACTATGGATGAACGAAATAGACAGATCGCTGAGATAGGTGCCTCACTG
ATTAAGCATTGGTAACTGTCAGACCAAGTTTACTCATATATACTTTAGATTGATTT
AAAACTTCATTTTTAATTTAAAAGGATCTAGGTGAAGATCCTTTTTGATAATCTCA
TGACCAAAATCCCTTAACGTGAGTTTTCGTTCCACTGAGCGTCAGACCCCGTAGA
AAAGATCAAAGGATCTTCTTGAGATCCTTTTTTTCTGCGCGTAATCTGCTGCTTGC
AAACAAAAAAACCACCGCTACCAGCGGTGGTTTGTTTGCCGGATCAAGAGCTACC
AACTCTTTTTCCGAAGGTAACTGGCTTCAGCAGAGCGCAGATACCAAATACTGTC
CTTCTAGTGTAGCCGTAGTTAGGCCACCACTTCAAGAACTCTGTAGCACCGCCTA
CATACCTCGCTCTGCTAATCCTGTTACCAGTGGCTGCTGCCAGTGGCGATAAGTC
GTGTCTTACCGGGTTGGACTCAAGACGATAGTTACCGGATAAGGCGCAGCGGTCG
GGCTGAACGGGGGGTTCGTGCACACAGCCCAGCTTGGAGCGAACGACCTACACC
GAACTGAGATACCTACAGCGTGAGCTATGAGAAAGCGCCACGCTTCCCGAAGGG
AGAAAGGCGGACAGGTATCCGGTAAGCGGCAGGGTCGGAACAGGAGAGCGCAC
GAGGGAGCTTCCAGGGGGAAACGCCTGGTATCTTTATAGTCCTGTCGGGTTTCGC
CACCTCTGACTTGAGCGTCGATTTTTGTGATGCTCGTCAGGGGGGCGGAGCCTAT
GGAAAAACGCCAGCAACGCGGCCTTTTTACGGTTCCTGGCCTTTTGCTGGCCTTTT
GCTCACATGTTCTTTCCTGCGTTATCCCCTGATTCTGTGGATAACCGTATTACCGC
CTTTGAGTGAGCTGATACCGGTCGCCGCAGCCGAACGACCGAGCGCAGCGAGTC
AGTGAGCGAGGAAGCGGAAGAGCGCCCAATACGCAAACCGCCTCTCCCCGCGCG
TTGGCCGATTCATTAATGCAGCTGGCACGACAGGTTTCCCGACTGGAAAGCGGGC
AGTGAGCGCAACGCAATTAATGTGAGTTACCTCACTCATTAGGCACCCCAGGCTT
TACACTTTATGCTTCCGGCTCCTATGTTGTGTGGAATTGTGAGCGGATAACAATTT

CACACAGGAAACAGCTATGACCATGATTACGCCAAGCTCGAAATACGACTCACT
ATAGGGCGAATTGGGTACCGGGCCGGCCGTCGAGCTTGATGGCATCGTGGTGTCA
CGCTCGTCGTTTGGTATGGCTTCATTCAGCTCCGGTTCCCAACGATCAAGGCGAGT
TACATGATCCCCCATGTTGTGAAAAAAGCGGTTAGCTCTTCGGTCCTCCGATCG
TTGTCAGAAGTAAGTTGGCCGCAGTGTTATCACTCATGGTTATGGCAGGAACTGC
ATAATTCTCTTACTGTCATGCCATCCGTAAGATGCTTTTCTGTGACTGGTGTACTC
AACCAAGTCATTCTGAGAATAGTGTATGCGGCGACCGAGTTGCTCTTGCCCGGCG
TCAACACGGGATAATACCGCGCCACATAGCAGAACTTTAAAAGTGCTCATCATTG
GAAAACGTTCTTCGGGGCGAAAACTCTCAAGGATCTTACCGCTGTTGAGATCCAG
TTCGATGTAACCCACTCGTGCACCCAACTGATCTTCAGCATCTTTTACTTTCACCA
GCGTTTCTGGGTGAGCAAAAACAGGAAGGCAAAATGCCGCAAAAAAGGGAATAA
GGGCGACACGGAAATGTTGAATACTCATACTCTTCCTTTTTCAATATTATTGAAGC
ATTTATCAGGGTTATTGTCTCATGAGCGATACATATTTGAATGTATTTAGAAAAAT
AAACAAATAGGGGTTCCGCGCACATTTCCCCGAAAAGTGCCACCTGACGTCTAAG
AAACCATTATTATCATGACATTAACCTATAAAAATAGGCGTATCACGAGGCCCTT
TCGTCTTCAAGAATTGGGGATCTACGTATGGTCATTCTTCTTCAGATTCCCTCATG
GAGAAGTGCGGCAGATGTATATGACAGAGTCGCCAGTTTCCAAGAGACTTTATTC
AGGCACTTCCATGATAGGCAAGAGAGAAGACCCAGAGATGTTGTTGTCCTAGTTA
CACATGGTATTTATTCCAGAGTATTCCTGATGAAATGGTTTAGATGGACATACGA
AGAGTTTGAATCGTTTACCAATGTTCCTAACGGGAGCGTAATGGTGATGGAACTG
GACGAATCCATCAATAGATACGTCCTGAGGACCGTGCTACCCAAATGGACTGATT
GTGAGGGAGACCTAACTACATAGTGTTTAAAGATTACGGATATTTAACTTACTTA
GAATAATGCCATTTTTTTGAGTTATAATAATCCTACGTTAGTGTGAGCGGGATTTA
AACTGTGAGGACCTCAATACATTCAGACACTTCTGACGGTATCACCCTACTTATTC
CCTTCGAGATTATATCTAGGAACCCATCAGGTTGGTGGAAGATTACCCGTTCTAA
GACTTTTCAGCTTCCTCTATTGATGTTACACTCGGACACCCCTTTTCTGGCATCCA
GTTTTTAATCTTCAGTGGCATGTGAGATTCTCCGAAATTAATTAAAGCAATCACAC
AATTCTCTCGGATACCACCTCGGTTGAAACTGACAGGTGGTTTGTTACGCATGCT
AATGCAAAGGAGCCTATATACCTTTGGCTCGGCTGCTGTAACAGGGAATATAAAG
GGCAGCATAATTTAGGAGTTTAGTGAACTTGCAACATTTACTATTTTCCCTTCTTA
CGTAAATATTTTTCTTTTTAATTCTAAATCAATCTTTTTCAATTTTTTGTTTGTATTC
TTTTCTTGCTTAAATCTATAACTACAAAAAACACATACAG

Sequence in red shows *PHO4*
Underlined sequences represent sites for primers.

### 4. Deletion of PHO80

- *Forward primer:* 
5’: CAGCGTATATTGGCTTTCCTTTAATCTAATGCCCCAAGCCCACAT
ACGATTTAGGTGACAC -3’
- *Backwards primer:*

5'- GGAGTTCTCAAGCTCATCTCGAAGTGTTTCTGTCGCTTATGAATACG
ACTCACTATAGGGTG -3'

Underlined sequences complement *hph* in pAG32 (Goldstein and McCusker, 1999,Yeast 15:1541-1553) The rest are deleting sequences that complement regions flanking *PHO80* (SGD). The plasmid pAG32 (Goldstein and McCusker, 1999), containing the selectable marker hygromycin B phosphotransferase *(hph),* was used as template. FW primer complements to non-coding strain. Starting at ∼ 483 bp upstream *hph* start (ATG) in pAG32 and 315 bp upstream *PHO80* start. BW primer complements to coding strand. Oligo. (63 bp.) starts 311 bp downstream *hph* end in pAG32, and 267 bp downstream *PHO80* end in S. cerevisiae.

**Resulting PCR product used for deletion of *PH080* by homologous recombination:**

CAGCGTATATTGGCTTTCCTTTAATCTAATGCCCCAAGCCACATACGATTTAGGT
GACACTATAGAACGCGGCCGCCAGCTGAAGCTTCGTACGCTGCAGGTCGACGGA
TCCCCGGGTTAATTAAGGCGCGCCAGATCTGTTTAGCTTGCCTCGTCCCCGCCGG
GTCACCCGGCCAGCGACATGGAGGCCCAGAATACCCTCCTTGACAGTCTTGACGT
GCGCAGCTCAGGGGCATGATGTGACTGTCGCCCGTACATTTAGCCCATACATCCC
CATGTATAATCATTTGCATCCATACATTTTGATGGCCGCACGGCGCGAAGCAAAA
ATTACGGCTCCTCGCTGCAGACCTGCGAGCAGGGAAACGCTCCCCTCACAGACGC
GTTGAATTGTCCCCACGCCGCGCCCCTGTAGAGAAATATAAAAGGTTAGGATTTG
CCACTGAGGTTCTTCTTTCATATACTTCCTTTTAAAATCTTGCTAGGATACAGTTCT
CACATCACATCCGAACATAAACAACCATGGGTAAAAAGCCTGAACTCACCGCGA
CGTCTGTCGAGAAGTTTCTGATCGAAAAGTTCGACAGCGTCTCCGACCTGATGCA
GCTCTCGGAGGGCGAAGAATCTCGTGCTTTCAGCTTCGATGTAGGAGGGCGTGGA
TATGTCCTGCGGGTAAATAGCTGCGCCGATGGTTTCTACAAAGATCGTTATGTTTA
TCGGCACTTTGCATCGGCCGCGCTCCCGATTCCGGAAGTGCTTGACATTGGGGAA
TTCAGCGAGAGCCTGACCTATTGCATCTCCCGCCGTGCACAGGGTGTCACGTTGC
AAGACCTGCCTGAAACCGAACTGCCCGCTGTTCTGCAGCCGGTCGCGGAGGCCAT
GGATGCGATCGCTGCGGCCGATCTTAGCCAGACGAGCGGGTTCGGCCCATTCGGA
CCGCAAGGAATCGGTCAATACACTACATGGCGTGATTTCATATGCGCGATTGCTG
ATCCCCATGTGTATCACTGGCAAACTGTGATGGACGACACCGTCAGTGCGTCCGT
CGCGCAGGCTCTCGATGAGCTGATGCTTTGGGCCGAGGACTGCCCCGAAGTCCGG
CACCTCGTGCACGCGGATTTCGGCTCCAACAATGTCCTGACGGACAATGGCCGCA
TAACAGCGGTCATTGACTGGAGCGAGGCGATGTTCGGGGATTCCCAATACGAGGT
CGCCAACATCTTCTTCTGGAGGCCGTGGTTGGCTTGTATGGAGCAGCAGACGCGC
TACTTCGAGCGGAGGCATCCGGAGCTTGCAGGATCGCCGCGGCTCCGGGCGTATA
TGCTCCGCATTGGTCTTGACCAACTCTATCAGAGCTTGGTTGACGGCAATTTCGAT
GATGCAGCTTGGGCGCAGGGTCGATGCGACGCAATCGTCCGATCCGGAGCCGGG
ACTGTCGGGCGTACACAAATCGCCCGCAGAAGCGCGGCCGTCTGGACCGATGGC
TGTGTAGAAGTACTCGCCGATAGTGGAAACCGACGCCCCAGCACTCGTCCGAGG
GCAAAGGAATAATCAGTACTGACAATAAAAAGATTCTTGTTTTCAAGAACTTGTC
ATTTGTATAGTTTTTTTATATTGTAGTTGTTCTATTTTAATCAAATGTTAGCGTGAT
TTATATTTTTTTTCGCCTCGACATCATCTGCCCAGATGCGAAGTTAAGTGCGCAGA
AAGTAATATCATGCGTCAATCGTATGTGAATGCTGGTCGCTATACTGCTGTCGATT
CGATACTAACGCCGCCATCCAGTGTCGAAAACGAGCTCGAATTCATCGATGATAT
CAGATCCACTAGTGGCCTATGCGGCCGCGGATCTGCCGGTCTCCCTATAGTGAGT
CGTATTCATAAGCGACAGAAAACACTTCGAGATGAGCTTGAGAACTCC

Nucleotides in blue shows the ORF of the *hgh* gene. Underlined sequences shows the PCR primers, partly complementary to the *PHO80* gene

## Claims

1. Process for preparing a *Saccharomyces cerevisiae* yeast strain having improved phytase activity, **characterized in that** the strain is modified by inserting one or more of the genes *PHO5, PHO10, PHO11* to overexpress said genes, and by deleting one or more of the regulatory, repressor genes *PHO80* and *PHO85,* whereby the insertion is either chromosomal or by transforming the strain using a plasmid containing said gene/s.

2. Process according to claim 1, wherein PHO80 has been deleted to form a mutant *pho80*Δ.

3. Process according to claim 1, wherein PHO85 has been deleted to form a mutant *pho85*Δ.

4. Process according to claims 1-2, wherein PHO5, PHO10 and PHO11 are overexpressed and PHO80 Is deleted.

5. A modified *Saccharomyces cerevisiae,* which contains an overexpression of one or more of the genes *PHO5, PHO10, PHO11,* and lacks one or more of regulatory, repressor genes *PHO80* and *PHO85* for increased phytase activity.

6. A modified *Saccharomyces cerevisiae* according to claim 5, wherein a regulating repressor gene, *PHO80* has been deleted to form a mutant *pho80*Δ.

7. A modified *Saccharomyces cerevisiae* according to one or more of claims 5-6, wherein a regulating repressor gene, *PHO85* has been deleted to form a mutant *pho85*Δ.

8. A modified *Saccharomyces cerevisiae* according to claim 5, wherein PHO5, PHO10 and PHO11 are overexpressed and PHO80 is deleted.

9. A modified *Saccharomyces cerevisiae* according to claims 5-8, wherein the strain contains a chromosomal integration of said gene/s or is transformed by a plasmid carrying said gene/s.

10. A method for the preparation of phytase, wherein one or more of the strains according to claims 5-9 are grown in a growth medium, whereupon any extracellularly produced phytase is isolated.

11. A method for preparing specific isomers of inositol phosphates and myo-Inositiol by using one or more of the strains according to claim 5-9 in a phytate and/or phytic acid containing growth medium.

12. A method according to claim 11, wherein I(1,2,4,5,6)P5 is produced and isolated.

13. Method according to claim 11, wherein I(1,2,5,6)P4 is produced and isolated.

14. Method according to claim 11, wherein I(1,2,6)P3 is produced and isolated.

15. Method according to claim 11, wherein I(1,2,)P2 is produced and isolated.

16. Method according to claim 11, wherein I(2)P1 is produced and isolated.

17. Method according to claim 11, wherein myo-inositiol is produced and isolated.

18. The use of one or more of the strains according to claims 5-9 as a live probiotic additive to foodstuff and feedstuff to obtain phytase activity in the gastrointestinal tract.

19. The use of one or more of the strains according to claims 5-9 in a yeast fermentation to degrade phytate during a fermentation process.

## Patentansprüche

1. Verfahren zur Herstellung eines *Saccharomyces cerevisiae-*Hefestammes mit verbesserter Phytase-Aktivität, **dadurch gekennzeichnet, dass** der Stamm durch Inserieren von einem oder mehreren der Gene PHO5, PHO10, PHO11 zur Überexpression dieser Gene und durch Deletion von einem oder mehreren der regulatorischen Repressorgene PHO80 und PHO85 modifiziert wird, wobei die Insertion entweder chromosomal oder durch Transformieren des Stammes unter Verwendung eines Plasmids mit einem Gehalt an diesem oder diesen Genen erfolgt.

2. Verfahren nach Anspruch 1, wobei PHO80 unter Bildung einer Mutante pho80Δ celetiert worden ist.

3. Verfahren nach Anspruch 1, wobei PHO85 unter Bildung einer Mutante pho85Δ deletiert worden ist.

4. Verfahren nach Anspruch 1-2, wobei PHO5, PHO10 und PHO11 überexprimiert werden und PHO80 deletiert wird.

5. Modifiziertes *saccharomyces cerevisiae,* das eine Überexpression von einem oder mehreren der Gene PHO5, PHO10, PHO11 enthält und dem eines oder mehrere der regulatorischen Repressorgene PHO80 und PHO85 fehlt, um eine erhöhte Phytase-Aktivität zu erzielen.

6. Modifiziertes *Saccharomyces cerevisiae* nach Anspruch 5, wobei ein regulierendes Repressorgen, PHO80, zur Bildung einer Mutante pho80Δ deletiert worden ist.

7. Modifiziertes *Saccharomyces cerevisiae* nach einem der Ansprüche 5-6, wobei ein regulierendes Repressorgen, PHO85, zur Bildung einer Mutante pho85Δ deletiert worden ist.

8. Modifiziertes *Saccharomyces cerevisiae* nach Anspruch 5, wobei PHO5, PHO10 und PHO11 überexprimiert sind und PHO80 deletiert ist.

9. Modifiziertes *Saccharomyces cerevisiae* nach den Ansprüchen 5-8, wobei der Stamm eine chromosomale Integration dieses Gens oder dieser Gene aufweist oder durch ein Plasmid, das dieses Gen oder diese Gene trägt, transformiert ist.

10. Verfahren zur Herstellung von Phytase, wobei einer oder mehrere der Stämme nach den Ansprüchen 5-9 in einem Wachstumsmedium gezüchtet werden, wonach extrazellulär gebildete Phytase isoliert wird.

11. Verfahren zur Ferstellung von spezifischen Isomeren von Inositphosphaten und Myoinosit unter Verwendung von einem oder mehreren der Stämme nach den Ansprüchen 5-9 in einem Phytat und/oder Phytinsäure enthaltenden Wachstumsmedium

12. Verfahren nach Anspruch 11, wobei I(1,2,4,5,6)P5 gebildet und isoliert wird.

13. Verfahren nach Anspruch 11, wobei I(1,2,5,6)P4 gebildet und isoliert wird.

14. Verfahren nach Anspruch 11, wobei I(1,2,6)P3 gebildet und isoliert wird.

15. Verfahren nach Anspruch 11, wobei I(1,2)P2 gebildet und isoliert wird.

16. Verfahren nach Anspruch 11, wobei I(2)P1 gebildet und isoliert wird.

17. Verfahren Anspruch 11, wobei Myoinosit gebildet und isoliert wird.

18. Verwendung von einem oder mehreren der Stämme nach den Ansprüchen 5-9 als lebendes probiotisches Additiv zu Nahrungs- und Futtermitteln, um im Magendarmtrakt Phytase-Aktivität zu erreichen.

19. Verwendung von einem oder mehreren der Stämme nach den Ansprüchen 5-9 zum Abbau von Phytat während eines Hefefermentationsprozesses.

## Revendications

1. Procédé de préparation d'une souche de levure *Saccharomyces cerevisiae* dotée d'une activité de phytase améliorée, **caractérisé en ce que** l'on modifie la souche en y insérant l'un ou plusieurs des gènes *PHO5*, *PHO10* et *PHO11* de telle sorte qu'il y ait surexpression dudit ou desdits gène(s), et en en délétant l'un ou plusieurs des gènes régulateurs répresseurs *PHO80* et *PHO85*, étant entendu qu'on réalise ladite insertion soit dans les chromosomes, soit en transformant la souche au moyen d'un plasmide contenant ledit ou lesdits gène(s).

2. Procédé conforme à la revendication 1, dans lequel on délète le gène *PHO80* pour obtenir un mutant *pho80Δ*.

3. Procédé conforme à la revendication 1, dans lequel on délète le gène *PHO85* pour obtenir un mutant *pho85Δ.*

4. Procédé conforme aux revendications 1 à 2, dans lequel les gènes *PHO5, PHO10* et *PHO11* sont surexprimés et le gène *PHO80* est délété.

5. Souche de *Saccharomyces cerevisiae* modifiée, chez laquelle il y a surexpression de l'un ou plusieurs des gènes *PHO5, PHO10* et *PHO11* et qui est dépourvue de l'un ou plusieurs des gènes régulateurs répresseurs *PHO80* et *PHO85,* ce qui lui confère une plus grande activité de phytase.

6. Souche de *Saccharomyces cerevisiae* modifiée, conforme à la revendication 5, chez laquelle le gène régulateur répresseur *PHO80* a été délété, ce qui donne un mutant *pho80Δ.*

7. Souche de *Saccharomyces cerevisiae* modifiée, conforme à l'une ou plusieurs des revendications 5 et 6, chez laquelle le gène régulateur répresseur *PHO85* a été délété, ce qui donne un mutant *pho85*Δ.

8. Souche de *Saccharomyces cerevisiae* modifiée, conforme à la revendication 5, chez laquelle les gènes *PHO5, PHO10* et *PHO11* sont surexprimés et le gène *PHO80* a été délété.

9. Souche de *Saccharomyces cerevisiae* modifiée, conforme à l'une des revendications 5 à 8, laquelle souche a intégré ledit ou lesdits gène(s) dans ses chromosomes ou a été transformée au moyen d'un plasmide porteur dudit ou desdits gène(s).

10. Procédé de production de phytase, dans lequel on cultive dans un milieu de croissance une ou plusieurs souches conformes aux revendications 5 à 9, après quoi l'on isole toute phytase produite dans le milieu extracellulaire.

11. Procédé de préparation de myo-inositol et d'isomères spécifiques de phosphates d'inositol, dans lequel on utilise une ou plusieurs souches conformes aux revendications 5 à 9, dans un milieu de croissance contenant un phytate et/ou de l'acide phytique.

12. Procédé conforme à la revendication 11, dans lequel on produit et isole du 1,2,4,5,6-pentaphosphate d'inositol.

13. Procédé conforme à la revendication 11, dans lequel on produit et isole du 1,2,5,6-tétraphosphate d'inositol.

14. Procédé conforme à la revendication 11, dans lequel on produit et isole du 1,2,6-triphosphate d'inositol.

15. Procédé conforme à la revendication 11, dans lequel on produit et isole du 1,2-diphosphate d'inositol.

16. Procédé conforme à la revendication 11, dans lequel on produit et isole du 2-monophosphate d'inositol.

17. Procédé conforme à la revendication 11, dans lequel on produit et isole du myo-inositol.

18. Utilisation d'une ou de plusieurs souches conformes aux revendications 5 à 9 en tant qu'adjuvant probiotique vivant ajouté à des aliments ou à de la nourriture pour animaux afin d'obtenir une activité de phytase dans le tractus gastro-intestinal.

19. Utilisation d'une ou de plusieurs souches conformes aux revendications 5 à 9 dans un procédé de fermentation par levure, afin de provoquer une dégradation des phylates au cours d'un tel procédé de fermentation.
